## Europäisches Patentamt
### European Patent Office
### Office européen des brevets

(11) Veröffentlichungsnummer: **0 288 965**
**A2**

(12) # EUROPÄISCHE PATENTANMELDUNG

(21) Anmeldenummer: 88106646.8

(22) Anmeldetag: 26.04.88

(51) Int. Cl.⁴: **C07K 5/00 , C07K 7/00 , A61K 37/02**

Patentansprüche für folgende Vertragsstaaten:
ES + GR.

(30) Priorität: 29.04.87 DE 3714277

(43) Veröffentlichungstag der Anmeldung:
**02.11.88 Patentblatt 88/44**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **König, Wolfgang, Dr.**
**Eppsteiner Strasse 25**
**D-6238 Hofheim am Taunus(DE)**
Erfinder: **Schindler, Peter, Dr.**
**Reinhardswaldweg 1**
**D-6082 Mörfelden-Walldorf(DE)**

(54) **Peptide mit Phospholipase A2- hemmender Wirkung.**

(57) Es werden Peptide mit Phospholipase $A_2$-hemmender Wirkung, deren C-terminale $\alpha$-Carboxylgruppe durch eine Ester-oder Amidfunktion geschützt ist oder zum Alkohol reduziert sein kann, beschrieben. Die Peptide der Formel L - B - A, worin L fehlen kann oder einen lipophilen Rest, B eine basische Aminosäure und A eine aromatische Aminosäure bedeuten, finden Verwendung als Heilmittel.

EP 0 288 965 A2

## Peptide mit Phospholipase A₂-hemmender Wirkung

Die Erfindung betrifft Peptide mit Phospholipase $A_2$-hemmender Wirkung, deren C-terminale $\alpha$-Carboxylgruppe durch eine Ester-oder Amidfunktion geschützt oder zum Alkohol reduziert sein kann.

Bekannte Peptid-artige Inhibitoren der Phospholipase $A_2$ sind die Lipocortine und Plipastatine. Lipocortine, die aus bis zu 346 Aminosäuren bestehen [B.P. Wallner et al., Nature 320 (1986) 77-81; K.-S. Huang et al., Cell 46 (1986) 191-199; C.J.M. Saris et al., Cell 46 (1986) 201-212], haben antigene Eigenschaften [F.R. Hirata et al., Proc. Natl. Acad. Sci. USA 78 (1981) 3190] und lassen deshalb einen therapeutischen Einsatz fraglich erscheinen. Die Plipastatine sind cyclische Dekapeptide, die von Bacillus cereus gebildet werden [T. Nishikiori et al., J. of Antibiotics 39 (1986) 755-761]. Die Aktivität der Plipastatine wurde nur für die aus Schweine-Pankreas isolierte Phospholipase $A_2$ beschrieben, deren Bedeutung für entzündliche Prozesse als nicht gesichert gilt.

Der Erfindung liegt daher die Aufgabe zugrunde, neue Peptide zu finden, die Phospholipase $A_2$ hemmen.

Diese Aufgabe wird erfindungsgemäß gelöst durch die Peptide mit Phospholipase $A_2$-hemmender Wirkung, deren C-terminale $\alpha$-Carboxylgruppe durch eine Esterfunktion oder eine Amidfunktion geschützt ist, der allgemeinen Formel I

L - B - A    (I)

in welcher

L    fehlen kann oder einen lipophilen Rest,

B    eine basische Aminosäure und

A    eine aromatische Aminosäure bedeuten,

sowie deren physiologisch verträgliche Salze.

Bevorzugt sind solche Reste L, B und A, die sich von natürlichen vorkommenden Aminosäuren (s. z.B. Schröder, Lübke, The Peptides, Volume I, New York 1965, Seiten 137-270), deren Antipoden oder deren einfachen Metaboliten ableiten.

Der Rest L steht insbesondere für eine N-terminal durch lipophile Acylreste, wie z.B. $(C_5-C_{14})$-Aryl-$(C_1-C_4)$-alkyloxycarbonyl, $(C_5-C_{14})$-Arylcarbonyl, $(C_1-C_{17})$-Alkylcarbonyl; oder durch Peptidreste, bestehend aus bis zu 4 natürlich vorkommenden Aminosäuren, geschützte lipophile Aminosäure in der L-oder D-Konfiguration, die acylartig an die $\alpha$-Aminogruppe der basischen Aminosäure gebunden ist oder für einen lipophilen Acylrest, wie z.B. $(C_5-C_{14})$-Aryl-$(C_1-C_4)$-alkyloxycarbonyl, insbesondere Benzyloxycarbonyl, $(C_5-C_{14})$-Aryl-$(C_1-C_4)$-alkylcarbonyl, insbesondere Indol-3-butyryl, $(C_5-C_{14})$-Arylcarbonyl, insbesondere Indol-2-carbonyl; oder einen $(C_1-C_{17})$-Alkylcarbonyl-Rest steht, sowie deren physiologische Salze.

Solche Verbindungen der Formel I sind bevorzugt, worin

L    Isoleucin, Leucin, Methionin, S-geschütztes-Cystein, Valin; gegebenenfalls durch Halogen, $(C_1-C_4)$-Alkyl oder $(C_1-C_4)$-Alkoxy substituiertes Phenylalanin, Tryptophan oder Naphthylalanin; an der Hydroxygruppe durch $(C_1-C_4)$-Alkyl substituiertes Tyrosin, Serin oder Threonin; am N des Imidazolringes durch $(C_1-C_4)$-Alkyl substituiertes Histidin; in der Seitenkette durch $(C_1-C_4)$-Alkyl veresterte oder durch liphophile Amine wie z.B. $(C_1-C_6)$-Alkylamine oder Cyclohexylamin amidierte Asparaginsäure, Glutaminsäure oder Aminoadipinsäure; oder an der $\omega$-Aminogruppe durch $(C_1-C_4)$-Alkylcarbonyl substituiertes bzw. unsubstituiertes Lysin oder Ornithin; lipophile Reste, wie z.B. Benzyloxycarbonyl, Indol-3-butyryl oder Indol-2-carbonyl, oder $(C_1-C_{17})$-Alkylcarbonyl (Fettsäurerest);

B    gegebenenfalls an der Guanidogruppe alkyliertes $(C_1-C_6)$-Arginin oder -Homoarginin, Lysin, Ornithin, Hydroxylysin oder Citrullin in der D-oder L-Konfiguration und

A    gegebenenfalls im Aromaten durch 1 bis 5 gleiche oder verschiedene Reste aus der Reihe Halogen, $(C_1-C_4)$-Alkyl oder $(C_1-C_4)$-Alkoxy substituiertes Phenylalanin, Tryptophan, Naphthylalanin, Thienylalanin oder Tetrahydroisochinolin-3-carbonsäure; oder gegebenenfalls an der Hydroxygruppe durch $(C_1-C_4)$-Alkyl substituiertes Tyrosin jeweils in der L-oder D-Konfiguration, deren Carboxylgruppen durch $(C_1-C_4)$-Alkyl-oder $(C_5-C_{14})$-Aryl-$(C_1-C_4)$-alkylester, durch die Amidgruppe, durch primäre $(C_1-C_4)$-Alkyl-, $(C_5-C_{14})$-Aryl-$(C_1-C_4)$-alkylamide und Peptidreste, bestehend aus bis zu 4 natürlich vorkommenden Aminosäuren, geschützt sind, bedeuten,

sowie deren physiologisch verträgliche Salze.

Als Salze kommen insbesondere Salze mit anorganischen oder organischen Säuren wie z.B. HCl, HBr,

$H_2SO_4$, $H_3PO_4$, Maleinsäure, Fumarsäure, Citronensäure, Weinsäure, Essigsäure in Frage. Die Chiralitätszentren in den neuen Peptiden können jeweils die R-, S-oder R,S-Konfiguration besitzen.

Alkyl kann geradkettig oder verzweigt sein. Entsprechendes gilt für davon abgeleitete Reste, wie z.B. Alkoxy und Alkylcarbonyl. Bevorzugt sind beispielsweise Methyl, Methoxy und Methanoyl.

Unter $(C_5-C_{14})$-Aryl ist auch Heteroaryl, wie z.B. Phenyl, Naphthyl, Biphenylyl, Fluorenyl, Pyrrol, Imidazol, Pyridin und Indol zu verstehen. Bevorzugt sind Phenyl und Indol. Entsprechendes gilt auch für davon abgeleitete Reste, wie z.B. Aralkyl, Aralkyloxycarbonyl und Arylcarbonyl.

Halogen bedeutet Fluor, Chlor, Brom oder Jod, vorzugsweise Chlor oder Brom.

$(C_5-C_{14})$-Aryl-$(C_1-C_4)$-alkylamide leiten sich bevorzugt von den biogenen Aminen, wie z.B. Phenethylamin, Tryptamin, Serotonin, p-Methoxyphenethylamin, 3,4-Dimethoxyphenethylamin, p-Hydroxyphenethylamin, 3,4-Dihydroxyphenethylamin oder Phenylalaninol ab.

L-B-A kann auch Bestandteil eines längeren Peptids oder eines Cyclopeptids sein, wobei die Struktur L-B-A teilweise oder ganz darin mehrmals vorkommen kann.

Die Erfindung betrifft weiterhin ein Verfahren zur Herstellung von Peptiden der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung in ihr physiologisch verträgliches Salz überführt.

Die Synthese der erfindungsgemäßen Peptide erfolgt nach den allgemeinen Methoden der Peptidchemie durch stufenweisen Aufbau vom C-terminalen Ende oder durch Segmentkondensation, wie sie z.B. in Wünsch et al., Houben-Weyl, Band 15/1 und 2 [Thieme, Stuttgart 1974] ausführlich beschrieben sind. Bei dem Verfahren mittels Segmentkupplung ist darauf zu achten, möglichst racemisierungsarme Kupplungsmethoden zu verwenden. In den hier vorliegenden Beispielen wurde zur Segmentkondensation vor allem die DCC/HOObt-Methode eingesetzt, die nach den bisherigen Erfahrungen mit die geringste Racemisierung während der Peptidsynthese liefert.

Die Phospholipase $A_2$ setzt Arachidonsäure aus Phospholipiden frei [G.J. Blackwell und R.J. Flower, Br. Med. Bull. 39 (1983) 260-264]. Da die Arachidonsäure über die Eicosanoid-Kaskade in Verbindungen wie Z.B. Prostaglandine und Leukotriene überführt wird, kann durch eine Hemmung der Phospholipase $A_2$ die Bildung dieser inflammatorisch wirksamen Arachidonsäure-Metabolite auf einer frühen Biosynthese-Stufe unterbunden werden. Die erfindungsgemäßen Phospholipase $A_2$-Inhibitoren sind daher insbesondere dort therapeutisch einsetzbar, wo Prostaglandine, z.B. vom $E_2$-Typ, Leukotriene, aber auch Thromboxane maßgeblich an der Pathogenese oder der Aufrechterhaltung pathophysiologischer Prozesse beteiligt sind. Hierzu gehören vor allem entzündliche, allergische, rheumatische und autoimmunologische Erkrankungen, wie z.B. Asthma bronchiale, Heuschnupfen, Arzneimittelallergie, Urtikaria, Hauterkrankungen wie Ekzeme und Dermatitiden, akutes rheumatisches Fieber, rheumatische Gelenkentzündungen, Muskelrheumatismus. Weitere Anwendungsgebiete sind Bluterkrankungen wie erworbene hämolytische Anämie, idiopathische Thrombopenie, Agranulozytose, Myeloblastose, Lymphogranulomatose, Hepatitis, Colitis ulcerosa, nephrotisches Syndrom, Hauterkrankungen wie Pemphigus, Lupus erythematodes, Dermatomyositis sowie die Behandlung von Abstoßungsreaktionen nach Transplantationen.

Die Erfindung betrifft daher auch die Verwendung von Peptiden der Formel I als Arzneimittel für oben angegebene Indikationen.

Getestet wurden die erfindungsgemäßen Peptide mit der Phospholipase $A_2$ aus polymorphonuklearen Leukozyten (PMN's). Da PMN's zu den Zelltypen gehören, die an inflammatorischen Prozessen wesentlich beteiligt sind [D.F. Bainton in G. Weissmann (Ed.) "The Cell Biology of Inflammation", Band 2, Seite 1 ff., Elsevier/North Holland Biomedical Press, Amsterdam 1980], kommt diesen Befunden eine besondere Bedeutung im Hinblick auf ihre therapeutische Anwendung zu. Als Enzym-Quelle wurden isolierte peritoneale Kaninchen-PMN's verwendet [Cunningham, J. Pathol. 128 (1979) 15-20], die in einer Konzentration von $3 \cdot 10^8$ Zellen/ml in destilliertem Wasser aufgenommen und als solche für den Test verwendet wurden. Die Bestimmung der Konzentration, bei der die Phospholipase $A_2$ durch die erfindungsgemäßen Verbindungen zu 50 % gehemmt wird ($IC_{50}$-Wert), wurde nach gängigen Methoden mit Hilfe einer Konzentrationsreihe ermittelt. Die Testsubstanzen wurden hierzu in 80 mmol Tris-HCl Puffer (pH 7,5) gelöst. Die Berechnung des $IC_{50}$-Wertes erfolgt mit Hilfe eines Computer-Programms entsprechend der Gleichung

$$\% \text{ Hemmung (beobachtet)} = \frac{100 \text{ \% Hemmung}}{1 + IC_{50}/[I]}$$

in der [I] die Konzentration des eingesetzten Inhibitors angibt.

**Phospholipase A₂-Hemmung**

| Beispiel Nr. | | IC$_{50}$ ($\mu$mol) |
|---|---|---|
| 1. | Z-Lys-Tyr-OMe | 530 |
| 2. | Z-Trp-Lys-Tyr-OMe | 8,5 |
| 3. | Z-Trp-Lys-Tyr-NH$_2$ | 10 |
| 4. | Z-Lys-Tyr-Phe-OMe | 11 |
| 5. | H-Lys-Tyr-Phe-OMe | 160 |
| 6. | Z-Trp-Lys-Tyr-Phe-OMe | 11 |
| 7. | Z-Lys-Tyr-Phe-NH$_2$ | 30 |
| 8. | Z-Trp-Lys-Tyr-Phe-NH$_2$ | 3,1 |
| 9. | H-Trp-Lys-Tyr-Phe-NH$_2$ | 26 |
| 10. | Z-D-Trp-Lys-Tyr-Phe-NH$_2$ | 33 |
| 11. | H-D-Phe-Cys-Phe-D-Trp-Lys-Tyr(Et)-Cys-Thr-ol | 12 |
| 12. | Z-Phe-Lys-Phe-Tyr-OMe | 37 |
| 13. | H-Lys-Phe-Leu-Lys-Phe-OtBu | 21 |
| 14. | Ac-Trp-Lys-Trp-NH-(CH$_2$)$_4$CH$_3$ | 13 |

|     |                                                              |        |
|-----|--------------------------------------------------------------|--------|
| 15. | H-D-Phe-Cys-Phe-D-Trp-Lys-Trp-Cys-Thr-ol (Cys–Cys bridge)    | 12     |
| 16. | H-Arg-Tyr(tBu)-OtBu                                           | 160    |
| 17. | Fmoc-Cys(StBu)-Arg-Phe-OtBu                                   | 41     |
| 18. | Fmoc-Arg-Pro-Cys(StBu)-Arg-Phe-OtBu                          | 5,2    |
| 19. | H-Arg-Pro-Cys(StBu)-Arg-Phe-OtBu                             | 24     |
| 20. | Z-Trp-Asp(OtBu)-Arg-Phe-NH$_2$                               | 16     |
| 21. | H-Arg-Trp-Asp(OtBu)-Arg-Phe-NH$_2$                          | 20     |
| 22. | Z-Asp(OtBu)-Arg-Trp-NH$_2$                                   | 39     |
| 23. | Z-Arg-Ile-Asp(OtBu)-Arg-Trp-NH$_2$                          | 13     |
| 24. | H-Arg-Ile-Asp(OtBu)-Arg-Trp-NH$_2$                          | 90     |
| 25. | Z-D-Phe-Trp-Lys-Tyr-Phe-OBzl                                 |        |
| 26. | cyclo-(D-Phe-Trp-Lys-Tyr-Phe)                                | 220    |
| 27. | Z-Lys-Lys-Tyr-Phe-OMe                                        | 69     |
| 28. | Fmoc-Arg-Tyr(tBu)-Phe-NH$_2$                                 | 30     |
| 29. | Fmoc-D-Arg-Tyr(tBu)-Phe-NH$_2$                               | 44     |
| 30. | Z-Trp-Arg-Tyr-Phe-NH$_2$                                     | 29     |
| 31. | Z-Trp-D-Arg-Tyr-Phe-NH$_2$                                   | 28     |
| 32. | Z-Trp-D-Lys-Tyr-Phe-NH$_2$                                   | 34     |
| 33. | Indolyl-3-butyryl-Trp-Lys-Tyr-Phe-NH$_2$                    | 6,1    |
| 34. | Indolyl-3-butyryl-Lys-Tyr-Phe-NH$_2$                        | 68     |
| 35. | Indolyl-2-carbonyl-Lys-Tyr-Phe-NH$_2$                       | 7,5    |
| 36. | H-Trp-Arg-Trp-Gly-Trp-Arg-Trp-Gly-OH                        | 2,4    |
| 37. | cyclo-(Trp-Arg-Trp-Gly-Trp-Arg-Trp-Gly)                     | 2,0    |
| 38. | Z-Trp-D-Lys-Tyr-Ile-Lys-Pro-OBzl                            | 7,2    |
| 39. | cyclo-(Trp-D-Lys-Tyr-Ile-Lys-Pro)                           | 69     |
| 40. | Z-Trp-Lys-Tyr-benzylamid                                     |        |
| 41. | Z-Trp-Lys-Tyr-phenethylamid                                  |        |
| 42. | Z-Trp-Lys-Tyr-Phe-ol                                         |        |
| 43. | Z-Trp-Lys-Tic-NH$_2$                                         |        |
| 44. | Z-D-Phe-D-Tyr-D-Lys-D-Trp-NH$_2$                            |        |

Appliziert werden die Substanzen hauptsächlich parenteral, nasal oder topisch. Doch auch eine p.o.-Applikation ist möglich, wenn durch geeignete Kapseln eine Zerstörung der empfindlichen Substanzen im Magen vermieden wird.

Beispiele

## 1. Z-Lys-Tyr-OMe

### 1a. Z-Lys(Boc)-Tyr(tBu)-OMe, $C_{33}H_{47}N_3O_8$ (MG 613,7)

13,95 g DCC (66 mmol) gibt man unter Rühren bei 0°C zu einer Lösung von 25,08 g (ca. 60-66 mmol) Z-Lys(Boc)-OH, 17,28 g (60 mmol) H-Tyr(tBu)-OMe·HCl und 7,78 ml N-Ethylmorpholin in 120 ml Dimethylformamid. Man läßt 2 Stunden bei 0°C und 6 Stunden bei Raumtemperatur rühren. Man läßt über Nacht bei Raumtemperatur stehen, saugt den Niederschlag ab und engt das Filtrat im Vakuum ein. Der Rückstand wird in Essigester gelöst und nacheinander mit Wasser, gesättiger NaHCO₃-Lösung, KHSO₄-Lösung, NaHCO₃-Lösung und Wasser geschüttelt. Die Essigesterphase wird über Na₂SO₄ getrocknet und eingeengt. Der Rückstand kristallisiert aus aus 200 ml Diisopropylether und 200 ml Petrolether.
Ausbeute 30,95 g, Schmp. 58-61°C, $[\alpha]_D^{21} = -6,8°$ (c = 1, Methanol).

### 1b. Z-Lys-Tyr-OMe-acetat, $C_{26}H_{34}N_3O_8$ (MG 516,57)

2,5 g Z-Lys(Boc)-Tyr(tBu)-OMe werden in 40 ml 90 %iger wäßriger Trifluoressigsäure gelöst. Man läßt 2 Stunden bei Raumtemperatur stehen und engt im Vakuum ein. Der Rückstand wird in Wasser gelöst und mit einem schwach basischen Ionenaustauscher in Acetat-Form verrrührt bis sich ein pH von 4-5 einstellt. Der Ionenaustauscher wird abfiltriert und das Filtrat wird gefriergetrocknet.
Ausbeute 1,87 g.
Zur Reinigung werden 800 mg der gewonnenen Substanz an einer Kieselgel-Säule in dem Laufmittel Methylenchlorid/Methanol/Essigsäure/Wasser wie 80:20:1:1 chromatographiert. Die einheitlichen Fraktionen werden vereinigt und im Vakuum eingeengt. Der Rückstand wird in Wasser gelöst und gefriergetrocknet.
Ausbeute 322 mg, $[\alpha]_D^{23} = +4,2°$ (c = 1, in 80 %iger wäßriger Essigsäure). Gehalt an Peptidbase laut Aminosäureanalyse: 88 %.

## 2. Z-Trp-Lys-Tyr-OMe

### 2a. H-Lys(Boc)-Tyr(tBu)-OMe·HCl, $C_{25}H_{42}Cl_1N_3O_6$ (MG 516,08)

12,28 g (20 mmol) Z-Lys(Boc)-Tyr(tBu)-OMe werden in Methanol gelöst und über Pd/C katalytisch mit Wasserstoff hydriert. Hierbei wird die freigesetzte Aminogruppe mit ca. 1 N methanolischer HCl mittels eines Autotitrators bei pH 4,5 titriert. Nach beendigter Reaktion wird der Katalysator abgesaugt und das Filtrat eingeengt. Der Rückstand wird mit Diethylether/Petrolether (1:1)-Behandlung fest und kann abgesaugt werden.
Ausbeute 9,31 g, Schmp. 150-152°C, $[\alpha]_D^{22} = +16,9°$ (c = 1, Methanol).

### 2b. Z-Trp-Lys(Boc)-Tyr(tBu)-OMe, $C_{44}H_{57}N_5O_9$ (MG 800)

Zu einer Lösung von 6,0 g (10 mmol) H-Lys(Boc)-Tyr(tBu)-OMe·HCl, 3,3 g (10 mmol) Z-Trp-OH und 1,86 g (10 mmol) HOBt in 50 ml Dimethylformamid gibt man bei 0°C unter Rühren 2,31 g DCC. Man verfährt nun wie bei Beispiel 1a. Der Rückstand wird aus Diethylether/Petrolether (1:1) kristallisiert.
Ausbeute 6,11 g.
Zur Reinigung wird an 200 g Kieselgel in Methylenchlorid/Methanol wie 95:5 chromatographiert.
Ausbeute 3,75 g, Schmp. 132°C, $[\alpha]_D^{21} = -12,9°$ (c = 1, in Methanol).

### 2c. Z-Trp-Lys-Tyr-OMe-acetat, $C_{37}H_{45}N_5O_9$ (MG 703,8)

1,5 g Z-Trp-Lys(Boc)-Tyr(tBu)-OMe werden in einer Mischung aus 20 ml 90 %iger wäßriger Trifluoressigsäure und 4 ml 1.2-Dimercaptoethan gelöst. Man läßt 1 Stunde bei Raumtemperatur stehen und engt im Vakuum ein. Der Rückstand wird in Wasser gelöst und mit einem schwach basischen Ionenaustauscher in Acetatform gerührt bis sich pH 3 eingestellt hat. Der Ionenaustauscher wird abfiltriert und das Filtrat gefriergetrocknet.
Ausbeute 0,86 g.

Zur Reinigung wird oben gewonnene Substanz an Kieselgel in einer Mischung aus 275 ml Wasser, 90,5 ml Essigsäure und 778 ml n-Butanol chromatographiert. Die einheitlichen Fraktionen werden vereinigt und im Vakuum eingeengt. Der Rückstand wird in Wasser gelöst und gefriergetrocknet.
Ausbeute 677 mg, $[\alpha]_D^{22} = -8,7°$ (c = 1, in 10%iger Essigsäure). Gehalt an Peptidbase laut Aminosäureanalyse: 87 %.

### 3. Z-Trp-Lys-Tyr-NH$_2$

### 3a. Z-Lys(Boc)-Tyr(tBu)-OH, $C_{32}H_{45}N_3O_8$ (MG 599,7)

Zu einer Lösung von 12,28 g (20 mmol) Z-Lys(Boc)-Tyr(tBu)-OMe in 75 ml Dioxan gibt man 25 ml Wasser und 21 ml 1N NaOH. Nach 2,5 Stunden wird der pH mit KHSO$_4$-Lösung auf pH 4 gestellt und im Vakuum zur Trockne eingeengt. Man verteilt den Rückstand zwischen einer KHSO$_4$/K$_2$SO$_4$-Lösung und Essigester. Die Essigesterphase wird mit Wasser ausgeschüttelt, über Na$_2$SO$_4$ getrocknet und eingeengt. Der Rückstand wird durch Verreiben mit Diisopropylether fest und kann abgesaugt werden.
Ausbeute 6,56 g, Schmp. 119-121°C unter Zersetzung, $[\alpha]_D^{22} = +3,3°$ (c = 1, Methanol).

### 3b. Z-Lys(Boc)-Tyr(tBu)-NH$_2$, $C_{32}H_{46}N_4O_7$ (MG 598,75)

Zu einer Lösung von 5,99 g (10 mmol) Z-Lys-(Boc)-Tyr(tBu)-OH und 1,52 g (10 mmol) HOBt·NH$_3$ in 100 ml Dimethylformamid gibt man unter Rühren bei 0°C 2,47 g (12 mmol) DCC. Man läßt 2 Stunden bei 0°C und 2 Stunden bei Raumtemperatur rühren und einen Tag bei Raumtemperatur stehen. Der Niederschlag wird abgesaugt und das Filtrat im Vakuum eingeengt. Der Rückstand wird mit gesättigter NaHCO$_3$-Lösung verrührt. Der Niederschlag wird abgesaugt, mit KHSO$_4$-Lösung und Wasser gewaschen und über P$_2$O$_5$ im Vakuum getrocknet.
Ausbeute 5,21 g.

Zur Reinigung wird die Substanz in 30 ml Methanol warm gelöst und mit 20 ml Wasser ausgefällt (Eisbad). Der Niederschlag wird abgesaugt und getrocknet.
Ausbeute 4,87 g.

Zur weiteren Reinigung wird die Substanz aus Essigester/Methyl-tert.-butylether umgefällt.
Ausbeute 4,3 g, Schmp. 153°C, $[\alpha]_D^{21} = -22,4°$ (c = 1, in Methanol).

### 3c. H-Lys(Boc)-Tyr(tBu)-NH$_2$·HCl, $C_{24}H_{41}Cl_1N_4O_5$ (MG 501,07)

3 g Z-Lys(Boc)-Tyr(tBu)-NH$_2$ werden analog Beispiel 2a katalytisch hydriert. Der Rückstand wird mit Diethylether verrieben und abgesaugt.
Ausbeute 2,14 g, Schmp. 187-189°C unter Zersetzung, $[\alpha]_D^{22} = +18,5°$ (c = 1, Methanol).

7

### 3d. Z-Trp-Lys(Boc)-Tyr(tBu)-NH$_2$, C$_{43}$H$_{56}$N$_6$O$_8$ (MG 784,9)

Zu einer Lösung von 1,35 g (4 mmol) Z-Trp-OH, 2,0 g (4 mmol) H-Lys(Boc)-Tyr(tBu)-NH$_2$·HCl, 0,57 g (4 mmol) HOBt und 0,52 ml (4 mmol) N-Ethylmorpholin in 25 ml Dimethylformamid gibt man unter Rühren bei 0°C 0,93 g (4,5 mmol) DCC. Man arbeitet wie bei Beispiel 1a auf. Der Rückstand wird in 20 ml Methanol gelöst und mit 20 ml Wasser gefällt. Der Niederschlag wird abgesaugt und im Vakuum über P$_2$O$_5$ getrocknet.
Ausbeute 2,05 g, Schmp. 158-160°C unter Zersetzung, $[\alpha]_D^{21}$ = -14,3° (c = 1, Methanol).

### 3e. Z-Trp-Lys-Tyr-NH$_2$-acetat, C$_{36}$H$_{44}$N$_6$O$_8$ (MG 688,78)

1,5 g Z-Trp-Lys(Boc)-Tyr(tBu)-NH$_2$ werden analog Beispiel 2c umgesetzt und gereinigt.
Ausbeute 851 mg, $[\alpha]_D^{22}$ = +1,5° (c = 1, 10%ige wäßrige Essigsäure). Gehalt an Peptidbase laut Aminosäureanalyse: 87 %.

### 4. Z-Lys-Tyr-Phe-OMe

### 4a. Z-Tyr(tBu)-Phe-OMe, C$_{31}$H$_{36}$N$_2$O$_6$ (MG 532,6)

Zu einer Lösung von 40,7 g Z-Tyr(tBu)-OH, 21,6 g H-Phe-OMe·HCl, 13,5 g HOBt und 13 ml N-Ethylmorpholin in 200 ml Dimethylformamid gibt man bei 0°C unter Rühren 22 g DCC. Man arbeitet wie im Versuch 1a auf. Der Rückstand wird mit Petrolether verrieben, abgesaugt und im Vakuum getrocknet.
Ausbeute 50,3 g (94,4 %), Schmp. 103-104°C, $[\alpha]_D^{24}$ = -12,3° (c = 1, in Methanol).

### 4b. H-Tyr(tBu)-Phe-OMe·HCl, C$_{23}$H$_{31}$Cl$_1$N$_2$O$_4$ (MG 434,9)

49,5 g Z-Tyr(tBu)-Phe-OMe werden in Methanol analog Beispiel 2a katalytisch hydriert.

Ausbeute 49 g einer schmierigen Substanz, die nicht kristallisierte.

### 4c. Z-Lys(Boc)-Tyr(tBu)-Phe-OMe, C$_{42}$H$_{56}$N$_4$O$_9$ (MG 760,9)

Zu einer Lösung von 21,5 g H-Tyr(tBu)-Phe-OMe·HCl, 6,67 g HOBt und 6,43 ml N-Ethylmorpholin in 150 ml Dimethylformamid gibt man bei 0°C unter Rühren 27,67 g Z-Lys(Boc)-OTcp. Man läßt eine Stunde bei 0°C und 2 Stunden bei Raumtemperatur rühren und engt ein. Aufgearbeitet wird analog Beispiel 1a. Der Rückstand wird in wenig Essigester gelöst und mit Petrolether gefällt. Der Niederschlag wird abgesaugt und im Vakuum getrocknet.
Ausbeute 29,1 g (77,4 %), Schmp. 93-94°C, $[\alpha]_D^{26}$ = -19,2° (c = 1, in Methanol).

### 4d. Z-Lys-Tyr-Phe-OMe, C$_{33}$H$_{40}$N$_4$O$_7$ (MG 604,7)

0,8 g Z-Lys(Boc)-Tyr(tBu)-Phe-OMe werden analog Beispiel 2c umgesetzt.
Ausbeute 0,44 g, $[\alpha]_D^{22}$ = -18,2° (c = 1, in Methanol). Gehalt an Peptidbase laut Aminosäureanalyse: 81 %.

### 5. H-Lys-Tyr-Phe-OMe

#### 5a. H-Lys(Boc)-Tyr(tBu)-Phe-OMe·HCl, $C_{43}H_{5\,1}Cl_1N_4O_7$ (MG 663,2)

28 g Z-Lys(Boc)-Tyr(tBu)-Phe-OMe werden analog Beispiel 2a katalytisch hydriert. Der Rückstand wird mit Diethylether verrieben, abgesaugt und im Vakuum getrocknet.
Ausbeute 22,4 g (92 %), Schmp. 145-147°C, $[\alpha]_D^{24} = +18,2°$ (c = 1, in Methanol).

#### 5b. H-Lys-Tyr-Phe-OMe-acetat, $C_{27}H_{38}N_4O_7$ (MG 530,6)

0,4 g H-Lys(Boc)-Tyr(tBu)-Phe-OMe·HCl werden analog Beispiel 2c umgesetzt.
Ausbeute 293 mg, $[\alpha]_D^{22} = +16,0°$ (c = 1, in Methanol). Gehalt an Peptidase laut Aminosäureanalyse: 66 %.

### 6. Z-Trp-Lys-Tyr-Phe-OMe

#### 6a. Z-Trp-Lys(Boc)-Tyr(tBu)-Phe-OMe, $C_{53}H_{66}N_6O_{10}$ (MG 947,16)

Zu einer Lösung von 1,58 g (4,7 mmol) Z-Trp-OH, 3,11 g (4,7 mmol) H-Lys(Boc)-Tyr(tBu)-Phe-OMe·HCl, 0,64 g (4,7 mmol) HOBt und 0,6 ml (4,7 mmol) N-Ethylmorpholin in 35 ml Dimethylformamid gibt man bei 0°C unter Rühren 1,07 g (5,2 mmol) DCC. Nun wird weiter verfahren wie in Beispiel 1a. Der Rückstand wird in Methanol gelöst und mit Wasser gefällt. Der Niederschlag wird abgesaugt und im Vakuum über $P_2O_5$ getrocknet.
Ausbeute 5,21 g, Schmp. 158-159°C, $[\alpha]_D^{19} = -25,5°$ (c = 1, in Dimethylformamid.

#### 6b. Z-Trp-Lys-Tyr-Phe-OMe-acetat, $C_{46}H_{54}N_6O_{10}$ (MG 850,9)

0,947 g (1 mmol) Z-Trp-Lys(Boc)-Tyr(tBu)-Phe-OMe werden analog Beispiel 2c umgesetzt. Der Rückstand wird mit Methyl-tert.-butyl-ether verrieben und abgesaugt. Die Verbindung wird aus Methanol umkristallisiert.
Ausbeute 0,28 g.
Die gesamte oben gewonnene Substanz wird in 1,5 ml Essigsäure gelöst, mit 150 ml Wasser verdünnt und gefriergetrocknet.
$[\alpha]_D^{20} = -1,7°$ (c = 1, in Wasser),
Gehalt an Peptidbase laut Aminosäureanalyse: 88 %.

### 7. Z-Lys-Tyr-Phe-NH₂

#### 7a. Z-Tyr-(tBu)-Phe-NH₂, $C_{10}H_{35}N_3O_5$ (MG 517,6)

Zu einer Lösung von 19,9 g (53 mmol) Z-Tyr(tBu)-OH, 10 g (50 mmol) H-Phe-NH₂·HCl, 6,8 g (50 mmol) HOBt und 6,4 ml (50 mmol) N-Ethylmorpholin in 75 ml Dimethylformamid gibt man bei 0°C unter Rühren 11,33 g (55 mmol) DCC. Man läßt 1 Stunde bei 0°C rühren und anschließend bei Raumtemperatur über Nacht stehen. Der Niederschlag wird abgesaugt und das Filtrat eingeengt. Der Rückstand wird mit gesättigter NaHCO₃-Lösung gerührt. Der Niederschlag wird abgesaugt und mit Wasser gewaschen. Die noch feuchte Substanz wird mit 150 ml Methanol aufgekocht und über Nacht kühl gestellt.
Ausbeute 23,1 g, Schmp. 182°C, $[\alpha]_D^{19} = -3,6°$ (c = 1, in Essigsäure).

### 7b. H-Tyr(tBu)-Phe-NH$_2$·HCl, C$_{22}$H$_{30}$Cl$_1$N$_3$O$_3$ (MG 419,96)

22,43 g Z-Tyr(tBu)-Phe-NH$_2$ werden analog Beispiel 2a katalytisch hydriert. Der Rückstand wird mit Diethylether verrieben.

Ausbeute 17,45 g, Schmp. 130-140°C unter Zersetzung, $[\alpha]_D^{19}$ = +36,1° (c = 1, in Essigsäure).

### 7c. Z-Lys(Boc)-Tyr(tBu)-Phe-NH$_2$, C$_{41}$H$_{55}$N$_5$O$_8$ (MG 745,9)

Zu einer Lösung von 16 g (42 mmol) Z-Lys(Boc)-OH, 16,76 g (40 mmol) H-Tyr(tBu)-Phe-NH$_2$·HCl, 6,56 g (40 mmol) HOObt und 5,12 ml (40 mmol) N-Ethylmorpholin in 100 ml Dimethylformamid gibt man bei 0°C unter Rühren 9,27 g (45 mmol) DCC. Der Ansatz wird analog Beispiel 7a aufgearbeitet. Umkristallisiert wird aus trockenem Ethanol.
Ausbeute 21,41 g, Schmp. 208°C, $[\alpha]_D^{19}$ = -34,7° (c = 1, in Dimethylformamid).

### 7d. Z-Lys-Tyr-Phe-NH$_2$, C$_{32}$H$_{39}$N$_5$O$_6$ (MG 589,7)

3,73 g (5 mmol) Z-Lys(Boc)-Tyr(tBu)-Phe-NH$_2$ werden analog Beispiel 2c umgesetzt. Der Rückstand wird in Wasser gelöst und mit NaHCO$_3$-Lösung auf pH 6 gestellt. Die wäßrige Lösung wird mit Essigester ausgeschüttelt und anschließend mit NaCl gesättigt. Nun tritt Kristallisation ein.
Ausbeute 2,41 g.
Aus absolutem Ethanol wird umkristallisiert.
Ausbeute 1,82 g, $[\alpha]_D^{22}$ = -9,7° (c = 1, in 80%iger wäßriger Essigsäure). Gehalt an Peptidbase laut Aminosäureanalyse: 52 %.

### 8. Z-Trp-Lys-Tyr-Phe-NH$_2$

### 8a. H-Lys(Boc)-Tyr(tBu)-Phe-NH$_2$·HCl, C$_{33}$H$_{49}$Cl$_1$N$_5$O$_6$ (MG 647,25)

14 g Z-Lys(Boc)-Tyr(tBu)-Phe-NH$_2$ werden analog Beispiel 2a katalytisch hydriert.
Ausbeute 10,7 g, 203-205° unter Zersetzung, $[\alpha]_D^{19}$ = +12,8° (c = 1, in Methanol).

### 8b. Z-Trp-Lys(Boc)-Tyr(tBu)-Phe-NH$_2$, C$_{52}$H$_{65}$N$_7$O$_9$ (MG 932,15)

Zu einer Lösung von 2,7 g (8 mmol) Z-Trp-OH, 5,1 g (8 mmol) H-Lys(Boc)-Tyr(tBu)-Phe-NH$_2$·HCl, 1,31 g (8 mmol) HOObt und 1,02 ml (8 mmol) N-Ethylmorpholin in 50 ml Dimethylformamid gibt man bei 0°C und unter Rühren 1,85 g (9 mmol) DCC. Man läßt 1 Stunde bei 0°C rühren und stellt über Nacht bei Raumtemperatur. Der Niederschlag wird abgesaugt und das Filtrat im Vakuum eingeengt. Der Rückstand wird in 100 ml Essigester gelöst. Dazu gibt man 100 ml Diethylether und stellt kalt. Der Niederschlag wird abgesaugt und im Vakuum getrocknet.
Ausbeute 6,11 g, Schmp. 186°C unter Zersetzung, $[\alpha]_D^{19}$ = -32,6° (c = 1, in Dimethylformamid).

### 8c. Z-Trp-Lys-Tyr-Phe-NH₂, C₄₃H₄₉N₇O₇ (MG 775,9)

2,0 g Z-Trp-Lys(Boc)-Tyr(tBu)-Phe-NH₂ werden analog Beispiel 2c umgesetzt. Der Rückstand wird mit Diethylether verrieben und abgesaugt. Die Substanz wird mit gesättigter NaHCO₃-Lösung verrieben und 1 Stunde gerührt. Der Niederschlag wird abgesaugt und aus Ethanol umkristallisiert.
Ausbeute 0,98 g, [α]$_D^{22}$ = -7,3° (c = 1, in 80%iger wäßriger Essigsäure). Gehalt an Peptidbase laut Aminosäureanalyse: 70 %.

### 9. H-Trp-Lys-Tyr-Phe-NH₂-diacetat, C₄₂H₅₁N₇O₉ (MG 797,9)

500 mg Z-Trp-Lys-Tyr-Phe-NH₂ werden in 90%iger wäßriger Essigsäure über Pd/C katalytisch hydriert. Nach beendigter Hydrierung wird der Katalysator abgesaugt und das Filtrat eingeengt. Der Rückstand wird mit Diethylether verrieben und abgesaugt. Anschließend wird die Substanz mit Ethanol verrührt, abgesaugt und mit einer Ethanol/Diethylether-Mischung gewaschen und im Vakuum getrocknet.
Ausbeute 300 mg, [α]$_D^{22}$ = +6,5° (c = 1, in 80 %iger Essigsäure). Gehalt an Peptidbase laut Aminosäureanalyse 74 %.

### 10. Z-D-Trp-Lys-Tyr-Phe-NH₂

### 10a. Z-D-Trp-Lys(Boc)-Tyr(tBu)-Phe-NH₂, C₅₂H₆₅N₇O₉ (MG 932,15)

Wird wie bei Beispiel 8b hergestellt.
Schmp. 192-194°C, [α]$_D^{19}$ = -21,3° (c = 1, in Dimethylformamid).

### 10b. Z-D-Trp-Lys-Tyr-Phe-NH₂, C₄₃H₄₉N₇O₇ (MG 775,9)

2 g Z-Trp-Lys(Boc)-Tyr(tBu)-Phe-NH₂ werden analog Beispiel 8c umgesetzt.
Ausbeute 0,98 g, [α]$_D^{22}$ = -7,3° (c = 1, in 80 %iger wäßriger Essigsäure), Gehalt an Peptidbase laut Aminosäureanalyse: 70 %.

## 11. H-D-Phe-Cys-Phe-D-Trp-Lys-Tyr(Et)-Cys-Thr-ol

### 11a. Z-D-Trp-Lys(Boc)-OH, C₃₀H₃₈N₄O₇ (MG 566,6)

Zu einer Suspension von 19,71 g H-Lys(Boc)-OH und 11,38 g HOBt in 160 ml Dimethylformamid gibt man bei Raumtemperatur unter Rühren 41,43 g Z-D-Trp-OTcp. Nach 4 Stunden Rühren bei Raumtemperatur ist alles gelöst. Man läßt über Nacht stehen und engt im Vakuum ein. Der Rückstand wird einer 3-stufigen Gegenstromverteilung in 400 ml-Portionen zwischen Essigester und halbgesättigter NaHCO₃-Lösung unterworfen. Die 3 Essigester-Fraktionen werden vereinigt und mit KHSO₄/K₂SO₄-Lösung und Wasser ausgeschüttelt, über Na₂SO₄ getrocknet und eingeengt. Der Rückstand wird mit Petrolether verrieben, abgesaugt und getrocknet.

Ausbeute 55,6 g.

Zur weiteren Reinigung wird die Substanz an 600 g Kieselgel chromatographiert. Zuerst wird nur mit Methylenchlorid eluiert, später mit einer Mischung aus Methylenchlorid/Methanol/Wasser wie 80:20:5.

Ausbeute 45,2 g, Schmp. 94°C unter Zersetzung, $[\alpha]_D^{24} = +0,7°$ (c = 1, in Methanol).

## 11b. H-D-Trp-Lys(Boc)-OH-acetat, $C_{24}H_{36}N_4O_7$ (MG 492,6)

41 g Z-D-Trp-Lys(Boc)-OH werden in 1500 ml 90 %iger wäßriger Essigsäure gelöst und über Pd/C mit Wasserstoff katalytisch hydriert. Nach beendigter Reaktion wird der Katalysator abgesaugt und das Filtrat eingeengt. Der Rückstand wird mit Ether verrieben, abgesaugt und im Vakuum getrocknet.

Ausbeute 32 g (89 %), $[\alpha]_D^{24} = -52,4°$ (c = 1, Methanol).

## 11c. Z-Phe-D-Trp-Lys(Boc)-OH·Cyclohexylamin, $C_{45}H_{60}N_6O_8$ (MG 813,0)

Zu einer Lösung von 31,53 g H-D-Trp-Lys(Boc)-OH-acetat und 9,11 g HOBt in 150 ml Dimethylformamid gibt man bei Raumtemperatur unter Rühren 25,37 g Z-Phe-ONSu. Man rührt 4 Stunden bei Raumtemperatur und läßt über Nacht bei Raumtemperatur stehen. Anderntags setzt man 400 ml Wasser und 64 ml gesättigte NaHCO₃-Lösung zu. Der Niederschlag wird abgesaugt und mit Wasser gut gewaschen und über $P_2O_5$ getrocknet.

Ausbeute 45,4 g (99,4 %)

Oben gewonnene Substanz wird zwischen Essigester, dem man etwas n-Butanol zusetzt und KHSO₄/K₂SO₄-Lösung verteilt. Die Essigester/n-Butanol-Phase wird über Na₂SO₄ getrocknet und eingeengt. Der Rückstand wird in Essigester gelöst und mit 7,3 ml Cyclohexylamin versetzt. Man stellt über Nacht bei 4°C und saugt den Niederschlag anderntags ab. Der Niederschlag wird noch einmal mit 350 ml Essigester aufgekocht, abgekühlt, abgesaugt und getrocknet.

Ausbeute 39,5 g (75,9 %), Schmp. 148-152°C unter Zersetzung, $[\alpha]_D^{22} = +9,6°$ (c = 1, in Methanol).

## 11d. H-Phe-D-Trp-Lys(Boc)-OH, $C_{31}H_{41}N_5O_6$ (MG 579,7)

39 g Z-Phe-D-Trp-Lys(Boc)-OH·Cyclohexylamin werden zwischen Essigester und 48 ml 1N $H_2SO_4$ unter Eiskühlung verteilt. Die Essigesterphase wird mit KHSO₄/K₂SO₄-Lösung und Wasser ausgeschüttelt, über Na₂SO₄ getrocknet und eingeengt. Der Rückstand wird in 600 ml 90 %iger Essigsäure gelöst und über Pd/C mit Wasserstoff katalytisch hydriert. Nach beendigter Reaktion wird der Katalysator abgesaugt und das Filtrat eingeengt. Der Rückstand wird mit Ether verrieben, abgesaugt und getrocknet.

Ausbeute 28,7 g.

Die Substanz wird aus Isopropanol umkristallisiert.

Ausbeute 21,85 g (78,6 %), Schmp. 142-144°C unter Zersetzung, $[\alpha]_D^{22} = +41°$ (c = 1, in Methanol).

## 11e. Fmoc-Cys(StBu)-Phe-D-Trp-Lys(Boc)-OH, $C_{53}H_{64}N_6O_9S_2$ (MG 993,3)

Zu einer Suspension von 5,8 g H-Phe-D-Trp-Lys(Boc)-OH und 1,42 g HOBt in 20 ml Dimethylformamid gibt man bei Raumtemperatur unter Rühren 6,11 g Fmoc-Cys(StBu)-OTcp. Man rührt 4 Stunden bei Raumtemperatur und läßt über Nacht bei Raumtemperatur stehen. Der Ansatz wird im Vakuum eingeengt und der Rückstand in Essigester gelöst und nacheinander mit Wasser, gesättigter NaHCO₃-Lösung, KHSO₄/K₂SO₄-Lösung und Wasser ausgeschüttelt, mit Na₂SO₄ getrocknet und eingeengt. Der Rückstand wird mit Petrolether verrieben und abgesaugt.

Ausbeute 8,18 g.

Zur Reinigung wird die oben gewonnene Substanz über 100 g Kieselgel chromatographiert. Als Elutionsmittel wird zunächst Methylenchlorid und dann eine Mischung aus Methylenchlorid/Methanol wie 80:20 verwendet. Die einheitlichen Fraktionen werden vereinigt, im Vakuum eingeengt und der Rückstand mit Petrolether verrieben und abgesaugt.

Ausbeute 6,42 g (64,6 %), Schmp. 1126-132°C unter Zersetzung, $[\alpha]_D^{25}$ = -68,4° (c = 1, in Methanol).

### 11f. Fmoc-Cys(StBu)-Thr-ol, $C_{26}H_{34}N_2O_5S_2$ (MG 518,7)

Zu einer Lösung von 5,36 g (20 mmol) H-Thr-ol•HOObt in 35 ml Dimethylformamid gibt an bei 0°C unter Rühren 12,2 g Fmoc-Cys(StBu)-OTcp. Man läßt 1 Stunde bei 0°C und 4 Stunden bei Raumtemperatur rühren, engt im Vakuum ein und löst den Rückstand in Essigester. Die Essigesterphase wird nacheinander mit Wasser, gesättigter $NaHCO_3$-Lösung, $KHSO_4/K_2SO_4$-Lösung und Wasser ausgeschüttelt, über $Na_2SO_4$ getrocknet und eingeengt. Die Substanz kristallisiert aus Isopropanol. Die Substanz wird abgesaugt und mit Petrolether gewaschen.

Ausbeute 6,63 g, Schmp. 155°C unter Zersetzung, $[\alpha]_D^{23}$ = -86,5° (c = 1, in Methanol).

### 11g. H-Cys(StBu)-Thr-ol•HOObt, $C_{18}H_{29}N_5O_5S_2$ (MG 459,6)

Zu einer Lösung von 6,47 g (12,5 mmol) Fmoc-Cys(StBu)-Thr-ol in 55 ml Dimethylformamid gibt man 12,87 ml (125 mmol) Diethylamin. Man läßt 5 Minuten bei Raumtemperatur stehen und engt im Vakuum ein. Der Rückstand wird in Methanol gelöst. Von Unlöslichem wird filtriert. Das Filtrat wird mit 2,03 g (12,5 mmol) HOObt versetzt und eingeengt. Der Rückstand wird mit Diethylether verrieben, abgesaugt und im Vakuum getrocknet.

Ausbeute 5,25 g, amorph, $[\alpha]_D^{23}$ = -26,3° (c = 1, in Methanol).

### 11h. Fmoc-Tyr(Et)-Cys(StBu)-Thr-ol, $C_{37}H_{48}N_3O_7S_2$ (MG 710,9)

Zu einer Lösung von 4,31 g (10 mmol) Fmoc-Tyr(Et)-OH und 4,58 g (10 mmol) H-Cys(StBu)-Thr-ol•HOObt in 40 ml Dimethylformamid gibt man bei 0°C unter Rühren 2,26 g (11 mmol) DCC. Es wird nun wie bei Beispiel 1a weiter verfahren. Der Rückstand wird mit Diethylether verrieben, abgesaugt und im Vakuum getrocknet.

Ausbeute 5,61 g.

Zur Reinigung chromatographiert man die Substanz an Kieselgel. Eluiert wird zuerst mit einer Mischung aus Methylenchlorid/Methanol wie 95:5 und später mit Methylenchlorid/Methanol wie 90:10. Die einheitlichen Fraktionen werden vereinigt und im Vakuum eingeengt. Der Rückstand wird mit Diethylether verrieben, abgesaugt und getrocknet.

Ausbeute 4,5 g, Schmp. 126°C, $[\alpha]_D^{23}$ = -73,4° (c = 1, in Methanol).

### 11i. H-Tyr(Et)-Cys(StBu)-Thr-ol, $C_{22}H_{36}N_3O_6S_2$ (MG 503,2)

Zu einer Lösung von 4,35 g (6 mmol) Fmoc-Tyr(Et)-Cys(StBu)-Thr-ol in 20 ml Dimethylformamid gibt man bei Raumtemperatur unter Rühren 6,2 ml (60 mmol) Diethylamin. Man läßt 5 Minuten bei Raumtemperatur rühren, engt ein und verreibt den Rückstand mit Diethylether.

Ausbeute 2,68 g (88,7 %), Schmp. 113°C, $[\alpha]_D^{22}$ = -69,8° (c = 1, in Methanol).

### 11k. Fmoc-Cys(StBu)-Phe-D-Trp-Lys(Boc)-Tyr(Et)-Cys(StBu)-Thr-ol, $C_{75}H_{99}N_9O_{13}S_4$ (MG 1462,7)

Zu einer Lösung von 4,26 g (4,3 mmol) Fmoc-Cys(StBu)-Phe-D-Trp-Lys(Boc)-OH, 2,16 g (4,3 mmol) H-Tyr(Et)-Cys(StBu)-Thr-ol und 0,72 g (4,3 mmol) HOObt in 15 ml Dimethylformamid gibt man bei 0°C unter Rühren 0,93 g (4,5 mmol) DCC und arbeitet analog Beispiel 7a weiter. Die Rohsubstanz wird mit Diethylether verrieben, abgesaugt, getrocknet und anschließend aus 60 ml Methanol und 30 ml Wasser umgefällt. Der Niederschlag wird abgesaugt und über $P_2O_5$ im Vakuum getrocknet.
Ausbeute 5,26 g (83,7 %).

**11l. H-Cys(StBu)-Phe-D-Trp-Lys(Boc)-Tyr(Et)-Cys(StBu)-Thr-ol, $C_{60}H_{89}N_9O_{11}S_4$ (MG 1240,5)**

Zu einer Lösung von 5,26 g (3,59 mmol) Fmoc-Cys(StBu)-Phe-D-Trp-Lys(Boc)-Tyr(Et)-Cys(StBu)-Thr-ol in 15 ml Dimethylformamid gibt man bei Raumtemperatur unter Rühren 14,36 ml (35,9 mmol) Diethylamin-Dimethylformamid-Mischung. Nach 5 Minuten wird im Vakuum eingeengt und der Rückstand mit Diethylether verrieben. Der Niederschlag wird abgesaugt und getrocknet.
Ausbeute 3,93 g, Schmp. 145-147°C unter Zersetzung, $[\alpha]_D^{22} = -81,8°$ (c = 1, in Dimethylformamid).

**11m. Boc-D-Phe-Cys(StBu)-Phe-D-Trp-Lys(Boc)-Tyr(Et)-Cys(StBu)-Thr-ol, $C_{74}H_{106}N_{10}O_{14}S_4$ (MG 1487,7)**

Zu einer Lösung von 0,87 g (3,3 mmol) Boc-D-Phe-OH, 3,7 g (3 mmol) H-Cys(StBu)-Phe-D-Trp-Lys-(Boc)-Tyr(Et)-Cys(StBu)-Thr-ol und 0,54 g (3,3 mmol) HOObt in 15 ml Dimethylformamid gibt man bei 0°C unter Rühren 0,72 g (3,5 mmol) DCC und arbeitet analog Beispiel 7a. Die Rohsubstanz wird aus 20 ml Methanol mit 20 ml Wasser gefällt.
Ausbeute 4,29 g, Schmp. 212-213°C unter Zersetzung, $[\alpha]_D^{22} = -66,2°$ (c = 1, in Dimethylformamid).

**11n. H-D-Phe-Cys-Phe-D-Trp-Lys-Tyr(Et)-Cys-Thr-ol-diacetat, $C_{60}H_{80}N_{10}O_{14}S_2$ (MG 1229,5)**

Zu einer Lösung von 4,16 g (2,9 mmol) Boc-D-Phe-Cys(StBu)-Phe-D-Trp-Lys(Boc)-Tyr(Et)-Cys(StBu)-Thr-ol in 50 ml Trifluorethanol gibt man 3,5 ml Tri-n-butylphosphin. Man rührt 4 Stunden bei Raumtemperatur und engt im Vakuum ein. Der Rückstand wird mit Essigester/Petrolether verrieben und abgesaugt.
Ausbeute 2,24 g.
Die oben gewonnene Substanz (1,71 mmol) werden in 150 ml Essigsäure gelöst und unter Rühren bei Raumtemperatur zu einer Mischung aus 1500 ml Essigsäure, 34,52 ml 0,1 N Jod-Lösung, 500 ml Wasser und 471 mg Natriumacetattrihydrat zugetropft. Man rührt 5 Minuten nach und entfärbt überschüssiges Jod mit wäßriger Ascorbinsäure-Lösung. Der Ansatz wird im Vakuum eingeengt und der Rückstand mit Wasser verrieben. Der Niederschlag wird abgesaugt, gut mit Wasser gewaschen und über $P_2O_5$ im Vakuum getrocknet.
Ausbeute 2,07 g.
Zur weiteren Reinigung werden 1 g der oben gewonnenen Substanz an Kieselgel in Methylenchlorid/Methanol wie 9:1 chromatographiert. Die einheitlichen Fraktionen werden vereinigt und im Vakuum eingeengt.
Ausbeute 680 mg.
Oben gewonnene 680 mg werden analog Beispiel 2c behandelt. Das rohe Diacetat (Ausbeute 451,3 mg) wird zur Reinigung durch Verteilungschromatographie an (R)Sephadex LH 20 chromatographiert. Hierbei wird (R)Sephadex LH 20 mit einer Mischung aus 275 ml Wasser, 90,5 ml Essigsäure und 778 ml n-Butanol gesättigt. Als Elutionsmittel dient eine Mischung aus 4300 ml Wasser, 430 ml n-Butanol und 350 ml Essigsäure.
Ausbeute 306 mg, $[\alpha]_D^{23} = -65,8°$ (c = 1, in 1 %iger Essigsäure).

**12. Z-Phe-Lys-Phe-Tyr-OMe**

14

**12a. Z-Phe-Tyr-OMe, $C_{27}H_{28}N_2O_5$ (MG 476,5)**

Zu einer Lösung von 14,95 g (50 mmol) Z-Phe-OH, 11,58 g (50 mmol) H-Tyr-OMe•HCl, 7 g (50 mmol) HOBt und 6,4 ml N-Ethylmorpholin in 150 ml Dimethylformamid gibt man bei 0°C und unter Rühren 11,33 g (55 mmol) DCC. Der Ansatz wird analog Beispiel 1a aufgearbeitet. Der Rückstand wird mit Petrolether verrieben.
Ausbeute 21,2 g (89 %), Schmp. 127°C, $[\alpha]_D^{21}$ = -15,1° (c = 1, in Methanol).

**12b. H-Phe-Tyr-OMe•HCl, $C_{19}H_{23}Cl_1N_2O_4$ (MG 378,8)**

9,52 g (20 mmol) Z-Phe-Tyr-OMe werden analog Beispiel 2a katalytisch hydriert. Der Rückstand wird mit Diethylether verrieben.
Ausbeute 7,05 g, Schmp. 128-131°C, $[\alpha]_D^{21}$ = +7,6° (c = 1, in Methanol.

**12c. Z-Lys(Boc)-Phe-Tyr-OMe, $C_{38}H_{48}N_4O_9$ (MG 704,8)**

Zu einer Lösung von 6,84 g (18 mmol) Z-LyS(Boc)-OH, 6,82 g (18 mmol) H-Phe-Tyr-OMe•HCl, 2,43 g (18 mmol) HOBt und 2,3 ml (18 mmol) N-Ethylmorpholin in 60 ml Dimethylformamid gibt man bei 0°C unter Rühren 4,2 g DCC. Der Ansatz wird analog Beispiel 1a aufgearbeitet. Der Rückstand wird mit Diethylether verrieben, abgesaugt und getrocknet.
Ausbeute 4,48 g, Schmp. 98°C, $[\alpha]_D^{21}$ = -9,2° (c = 1, in Methanol).

**12d. H-Lys(Boc)-Phe-Tyr-OMe•HCl, $C_{30}H_{43}Cl_1N_4O_7$ (MG 607,16)**

4,3 g Z-Lys(Boc)-Phe-Tyr-OMe werden analog Beispiel 2a katalytisch hydriert. Der Rückstand wird mit Diethylether verrieben.
Ausbeute 3,65 g, Schmp. 132°C, $[\alpha]_D^{21}$ = -4,0° (c = 1, in Methanol).

**12e. Z-Phe-Lys(Boc)-Phe-Tyr-OMe, $C_{47}H_{57}N_5O_{10}$ (MG 852,0)**

Zu einer Lösung von 1,79 g (6 mmol) Z-Phe-OH, 3,64 g (6 mmol) H-Lys(Boc)-Phe-Tyr-OMe•HCl, 0,81 g (6 mmol) HOBt und 0,77 ml N-Ethylmorpholin in 35 ml Dimethylformamid gibt man bei 0°C unter Rühren 1,35 g (6,6 mmol) DCC. Der Ansatz wird analog Beispiel 7a aufgearbeitet. Die Rohsubstanz wird in 30 ml Methanol gelöst und mit 30 ml Wasser gefällt.
Ausbeute 6,19 g, Schmp. 151°C, $[\alpha]_D^{21}$ = -10,6° (c = 1, in Dimethylformamid).

**12f. Z-Phe-Lys-Phe-Tyr-OMe-acetat $C_{44}H_{53}N_5O_{10}$ (MG 811,9)**

0,852 g (1 mmol) Z-Phe-Lys(Boc)-Phe-Tyr-OMe werden analog Beispiel 2c behandelt. Der Rückstand wird mit Methyl-tert.-butyl-ether verrieben und anschließend aus Methanol/Wasser umkristallisiert.
Ausbeute 0,34 g.
Die oben gewonnene Substanz wird in 2 ml Essigsäure gelöst. Die Lösung wird mit 150 ml Wasser versetzt und gefriergetrocknet.
Ausbeute 231 mg, Schmp. 205°C, $[\alpha]_D^{21}$ = -19,8° (c = 1, in Dimethylformamid)
Aminosäureanalyse: Phe 2,0, Tyr 0,78, Lys 1,0; Gehalt an Peptidbase laut Aminosäureanalyse: 91 %

### 13. H-Lys-Phe-Leu-Lys-Phe-OtBu

#### 13a. Fmoc-Lys(Z)-Phe-OtBu, $C_{42}H_{47}N_3O_7$ (MG 705,86)

Zu einer Lösung von 5,03 g (10 mmol) Fmoc-Lys(Z)-OH, 2,6 g (10 mmol) H-Phe-OtBu·HCl, 1,35 g (10 mmol) HOBt und 1,3 ml (10 mmol) N-Ethylmorpholin in 50 ml Dimethylformamid gibt man bei 0°C unter Rühren 2,2 g DCC. Der Ansatz wird analog Beispiel 1a aufgearbeitet. Der Rückstand wird mit Diethylether verrieben, abgesaugt und getrocknet.
Ausbeute 6,2 g, $[\alpha]_D^{22} = -6,74°$ (c = 1, in 90 %iger wäßriger Essigsäure).

#### 13b. Fmoc-Lys(Z)-Phe-OH, $C_{38}H_{39}N_3O_7$ (MG 649,75)

2,95 g Fmoc-Lys(Z)-Phe-OtBu werden in 30 ml 90 %iger wäßriger Trifluoressigsäure gelöst. Man läßt 1 Stunde bei Raumtemperatur stehen und engt im Vakuum ein. Der Rückstand wird mit Diethylether verrieben und abgesaugt.
Ausbeute 2,32 g.

#### 13c. Fmoc-Leu-Lys(Z)-Phe-OtBu, $C_{48}H_{58}N_4O_8$ (MG 819,02)

Zu einer Lösung von 3,17 g (4,5 mmol) Fmoc-Lys(Z)-Phe-OtBu in 20 ml Dimethylformamid gibt man 4,7 ml (45 mmol) Diethylamin. Man rührt 10 Minuten bei Raumtemperatur und engt im Vakuum ein. Der Rückstand wird in wenig Methylenchlorid gelöst filtriert und chromatographisch an Kieselgel gereinigt. Als Elutionsmittel verwendet man zunächst Methylenchlorid um die Verunreinigungen herauszulösen und später zur Elution des Tripeptids eine Mischung aus Methylenchlorid/Methanol wie 95:5.
Ausbeute 2,3 g ölige Substanz.
Zu einer Lösung von 1,58 g (4,5 mmol) Fmoc-Leu-OH, oben gewonnene 2,3 g H-Leu-Lys(Z)-Phe-OtBu und 607,5 mg HOBt in 20 ml Dimethylformamid gibt man bei 0°C unter Rühren 940 ml DCC. Der Ansatz wird analog Beispiel 1a aufgearbeitet. Der Rückstand wird mit Diethylether verrieben, abgesaugt und im Vakuum getrocknet.
Ausbeute 2,7 g.

#### 13d. Fmoc-Lys(Z)-Phe-Leu-Lys(Z)-Phe-OtBu, $C_{71}H_{58}N_7O_{13}$ (MG 1244,5)

Zu einer Lösung von 2,67 g (3,26 mmol) Fmoc-Leu-Lys(Z)-Phe-OtBu in 20 ml Dimethylformamid gibt man bei Raumtemperatur 3,42 ml (32,6 mmol) Diethylamin und rührt 10 Minuten bei Raumtemperatur. Man arbeitet analog Beispiel 13c auf.
Ausbeute 2 g ölige Substanz.
Zu einer Lösung von 2,2 g (3,27 mmol) Fmoc-Lys(Z)-Phe-OH, 2 g oben gewonnenes Öl und 533 g HOObt in 20 ml Dimethylformamid gibt man bei 0°C 720 g DCC. Der Ansatz wird analog Beispiel 7a aufgearbeitet.
Ausbeute 3,14 g, Schmp. 178-182°C, $[\alpha]_D^{22} = -20,8°$ (c = 1, in 90 %iger wäßriger Essigsäure.

#### 13e. H-Lys-Phe-Leu-Lys-Phe-OtBu-triacetat, $C_{45}H_{75}N_7O_{12}$ (MG 918,1)

1 g Fmoc-Lys(Z)-Phe-Leu-Lys(Z)-Phe-OtBu werden in 20 ml 90 %iger wäßriger Essigsäure gelöst und über Pd/C katalytisch hydriert. Nach beendigter Reaktion wird der Katalysator abgesaugt und das Filtrat eingeengt. Der Rückstand wird mit Diethylether verrieben und abgesaugt.
Ausbeute 750 mg.

Die Substanz wird an Kieselgel chromatographisch gereinigt. Als Elutionsmittel dient eine Mischung aus 450 ml Methylenchlorid, 200 ml Methanol, 150 ml Methylglykol, 100 ml Wasser und 5 g Ammoniumacetat. Ausbeute 450 mg, $[\alpha]_D^{23}$ = -25,5° (c = 1, in Wasser), Aminosäureanalyse: Leu 1,0; Phe 1,96; Lys 2,03; Gehalt an Peptidbase laut Aminosäureanalyse: 62 %.

## 14. Ac-Trp-Lys-Trp-NH-(CH$_2$)$_4$-CH$_3$

### 14a. Z-Trp-n-pentylamid, C$_{24}$H$_{29}$N$_3$O$_3$ (MG 407,5)

Zu einer Lösung von 16,9 g (50 mmol) Z-Trp-OH, 4,35 g (50 mmol) n-Pentylamin und 7 g HOBt in 100 ml Dimethylformamid gibt man bei 0°C unter Rühren 11,33 g (55 mmol) DCC. Man arbeitet analog Beispiel 1a auf. Der Rückstand kristallisiert aus Diethylether/Petrolether wie 1:1.
Ausbeute 18,35 g (90 %), Schmp. 146°C, $[\alpha]_D^{21}$ = -22,3° (c = 1, in Dimethylformamid).

### 14b. H-Trp-n-pentylamid•HCl, C$_{16}$H$_{24}$Cl$_1$N$_3$O$_1$ (MG 309,85)

16,28 g (40 mmol) Z-Trp-n-pentylamid werden analog Beispiel 2a katalytisch hydriert. Der Rückstand kristallisiert aus Diethylether/Petrolether.
Ausbeute 12,28 g (99 %), Schmp. 152°C, $[\alpha]_D^{21}$ = +44,1° (c = 1, in Methanol).

### 14c. Z-Lys(Boc)-Trp-n-pentylamid, C$_{35}$H$_{49}$N$_5$O$_6$ (MG 635,8)

Zu einer Lösung von 6,2 g (20 mmol) H-Trp-n-pentylamid•HCl, 7,6 g (20 mmol) Z-Lys(Boc)-OH, 2,7 g HOBt und 2,56 ml (20 mmol) N-Ethylmorpholin in 70 ml Dimethylformamid gibt man bei 0°C unter Rühren 4,73 g (23 mmol) DCC. Der Ansatz wird analog Beispiel 1a aufgearbeitet. Die Substanz kristallisiert aus Diethylether/Petrolether.
Ausbeute 11 g (86,6 %), Schmp. 126°C, $[\alpha]_D^{21}$ = -14,1° (c = 1, in Dimethylformamid).

### 14d. H-Lys(Boc)-Trp-n-pentylamid•HCl, C$_{27}$H$_{44}$Cl$_1$N$_5$O$_4$ (MG 538,1)

9,52 g (15 mmol) Z-Lys(Boc)-Trp-n-pentylamid werden analog Beispiel 2a katalytisch hydriert. Der Rückstand kristallisiert aus Diethylether.
Ausbeute 5,91 g, Schmp. 100-128°C unter Zersetzung, $[\alpha]_D^{21}$ = +23,6° (c = 1, in Methanol).

### 14e. Z-Trp-Lys(Boc)-Trp-n-pentylamid, C$_{46}$H$_{59}$N$_7$O$_7$ (MG 822)

Zu einer Lösung von 5.38 g (10 mmol) H-Lys(Boc)-Trp-n-pentylamid•HCl, 3,38 g (10 mmol) Z-Trp-OH, 1,4 g (10 mmol) HOBt und 1,28 ml (10 mmol) N-Ethylmorpholin in 40 ml Dimethylformamid gibt man bei 0°C unter Rühren 2,47 g (12 mmol) DCC. Der Ansatz wird analog Beispiel 1a aufgearbeitet. Die Rohsubstanz wird aus Ethanol/Wasser umgefällt, abgesaugt und über P$_2$O$_5$ getrocknet.
Ausbeute 5,63 g, Schmp. 188-190°C unter Zersetzung, $[\alpha]_D^{21}$ = -22,7° (c = 1, in Dimethylformamid).

### 14f. H-Trp-Lys(Boc)-Trp-n-pentylamid·HCl, $C_{38}H_{54}Cl_1N_7O_5$ (MG 724,36)

3,7 g (4,5 mmol) Z-Trp-Lys(Boc)-Trp-n-pentylamid werden analog Beispiel 2a katalytisch hydriert. Der Rückstand kristallisiert aus Diethylether.
Ausbeute 3,04 g (93 %), Schmp. 200-202°C unter Zersetzung, $[\alpha]_D^{21} = +4,2°$ (c = 1, in Methanol).

### 14g. Ac-Trp-Lys(Boc)-Trp-n-pentylamid, $C_{40}H_{44}N_6O_6$ (MG 704,84)

Zu einer Lösung von 2,89 g (4 mmol) H-Trp-Lys(Boc)-Trp-n-pentylamid·HCl und 0,51 ml (4 mmol) N-Ethylmorpholin in 25 ml Dimethylformamid gibt man bei 0°C unter Rühren 0,7 g Essigsäure-N-hydroxysuccinimidester. Man läßt über Nacht bei Raumtemperatur stehen und engt ein. Der Rückstand wird mit Wasser verrieben, abgesaugt und über $P_2O_5$ getrocknet. Die Substanz wird in der Hitze in 75 ml Methanol gelöst und in der Hitze mit 40 ml Wasser versetzt. Man kühlt auf 0°C ab. Hierbei kristallisiert die Substanz aus.
Ausbeute 2,52 g, Schmp. 192-194°C unter Zersetzung, $[\alpha]_D^{21} = -20,3°$ (c = 1, in Dimethylformamid).

### 14h. Ac-Trp-Lys-Trp-n-pentylamid-triacetat, $C_{41}H_{59}N_7O_{10}$ (MG 809,9)

1 g Ac-Trp-Lys(Boc)-Trp-n-pentylamid werden analog Beispiel 2c umgesetzt. Der Rückstand wird mit Methyl-tert.-butylether verrieben und abgesaugt. Danach wird die Substanz mit NaHCO$_3$-Lösung verrieben, abgesaugt und mit Wasser gewaschen. Die so gewonnene Substanz wird in 50 ml 3 %iger Essigsäure gelöst. Von Unlöslichem wird abfiltriert und das Filtrat gefriergetrocknet.
Ausbeute 798,3 mg, $[\alpha]_D^{20} = +21,5°$ (c = 1, in Wasser), Gehalt an Peptidbase laut Aminosäureanalyse: 71 %.

## 15. H-D-Phe-Cys-Phe-D-Trp-Lys-Trp-Cys-Thr-ol

### 15a. Fmoc-Trp-Cys(StBu)-Thr-ol, $C_{37}H_{44}N_45O_6S_2$ (MG 704,9)

Zu einer Lösung von 2,13 g Fmoc-Trp-OH und 2,3 g H-Cys(StBu)-Thr-ol·HOObt (siehe Beispiel 11g) in 15 ml Dimethylformamid gibt man bei 0°C, 1,13 g DCC. Der Ansatz wird analog Beispiel 1a aufgearbeitet. Der Rückstand wird mit Petrolether verrieben, abgesaugt und im Vakuum getrocknet.
Ausbeute 3,13 g.
Zur Reinigung wird die Substanz 30 Minuten mit Diethylether verrührt, abgesaugt und getrocknet.
Ausbeute 2,23 g (63 %), Schmp. 101-111°C, $[\alpha]_D^{25} = -77,5°$ (c = 1, Methanol).

### 15b. Fmoc-Cys(StBu)-Phe-D-Trp-Lys(Boc)-Trp-Cys(StBu)-Thr-ol, $C_{75}H_{96}N_{12}O_{12}S_4$ (MG 1485,95)

Zu einer Lösung von 1,97 g (2,8 mmol) Fmoc-Trp-Cys(StBu)-Thr-ol in 28 ml Dimethylformamid gibt man bei Raumtemperatur unter Rühren 12,2 ml (28 mmol) Diethylamin-Dimethylformamid-Lösung. Man rührt 5 Minuten bei Raumtemperatur und engt im Vakuum ein. Der Rückstand wird analog Beispiel 13c über 60 g Kieselgel chromatographiert.
Ausbeute 0,9 g (66,6 %) ölige Substanz.
Zu einer Lösung der oben gewonnenen 0,9 g H-Trp-Cys(StBu)-Thr-ol, 1,85 g Fmoc-Cys(StBu)-Phe-D-Trp-Lys(Boc)-OH (siehe Beispiel 11e) und 0,31 g HOObt in 6 ml Dimethylformamid gibt man bei 0°C unter Rühren 0,41 g DCC. Der Ansatz wird analog Beispiel 7a aufgearbeitet.

Ausbeute 2,58 g (93,4 %).

Zur Reinigung wird die oben gewonnene Substanz an Kieselgel chromatographiert. Eluiert wird mit einer Mischung aus Methylenchlorid/Methanol wie 92 : 8. Die einheitlichen Fraktionen werden im Vakuum eingeengt und der Rückstand mit Petrolether verrieben.

Ausbeute 1,8 g (65,2 %), Schmp. 180-182°C unter Zersetzung, $[\alpha]_D^{21} = -113,9°$ (c = 1, in Methanol).

### 15c. H-Cys(StBu)-Phe-D-Trp-Lys(Boc)-Trp-Cys(StBu)-Thr-ol, $C_{60}H_{86}N_{10}O_{10}S_{44}$ (MG 1235,7)

Zu einer Lösung von 1,64 g Fmoc-Cys(StBu)-Phe-D-Trp-Lys(Boc)-Trp-Cys(StBu)-Thr-ol (1,1 mmol) in 11 ml Dimethylformamid gibt man 4,4 ml (11 mmol) Diethylamin-Dimethylformamid-Lösung. Man rührt 5 Minuten bei Raumtemperatur und engt im Vakuum ein. Der Rückstand wird analog Beispiel 13c an Kieselgel chromatographiert. Die einheitlichen Fraktionen werden vereinigt, im Vakuum eingeengt und mit Petrolether verrieben.

Ausbeute 0,74 g (54,4 %), Schmp. 144-148°C, $[\alpha]_D^{25} = -119,9°$ (c = 1, in Methanol).

### 15d. H-D-Phe-Cys-Phe-D-Trp-Lys-Trp-Cys-Thr-ol-diacetat, $C_{60}H_{77}N_{11}O_{13}S_2$ (MG 1224,47)

Zu einer Lösung von 0,41 g Boc-D-Phe-OH, 0,62 g H-Cys(StBu)-Phe-D-Trp-Lys(Boc)-Trp-Cys(StBu)-Thr-ol und 0,071 g HOBt in 3 ml Dimethylformamid gibt man bei 0°C unter Rühren 0,11 g DCC. Der Ansatz wird analog Beispiel 7a aufgearbeitet.

Ausbeute 0,74 g.

Zur Reinigung wird die oben gewonnene Substanz an Kieselgel chromatographiert. Eluiert wird mit einer Mischung aus Methylenchlorid/Methanol wie 93:7.

Ausbeute 350 mg.

Zu einer Lösung von 0,34 g (0,23 mmol) des oben gewonnenen Boc-D-Phe-Cys(StBu)-Phe-D-Trp-Lys-(Boc)-Trp-Cys(StBu)-Thr-ol in 5 ml Trifluorethanol gibt man bei Raumtemperatur 0,58 ml (2,3 mmol) Tri-n-butylphosphin. Man rührt 4 Stunden bei Raumtemperatur und engt ein. Der Rückstand wird mit einer Mischung aus Essigester/Petrolether verrieben und abgesaugt.

Ausbeute 290 mg.

281 mg des oben gewonnenen Boc-D-Phe-Cys-Phe-D-Trp-Lys(Boc)-Trp-Cys-Thr-ol werden in Eisessig gelöst. Diese Lösung läßt man unter Rühren bei Raumtemperatur schnell zu einer Mischung aus 215 ml Eisessig, 71,6 ml Wasser, 58,8 ml Natriumacetat und 4,4 ml 0,1N Jod-Lösung zutropfen. Man läßt noch 5 Minuten nachrühren und entfärbt die Lösung mit einer wäßrigen Ascorbinsäure-Lösung. Die Lösung wird eingeengt und der Rückstand mit Wasser verrieben, abgesaugt und über $P_2O_5$ getrocknet. Die Substanz wird an Kieselgel chromatographiert. Eluiert wird mit einer Mischung aus Methylenchlorid/Methanol wie 95:5. Die eingeengten Fraktionen werden mit Petrolether verrieben.

Ausbeute 120 mg.

110 mg des oben gewonnenen

### Boc-D-Phe-Cys-Phe-D-Trp-Lys(Boc)-Trp-Cys-Thr-ol

werden analog Beispiel 2c umgesetzt. Der Rückstand wird zwischen Essigester und Wasser verteilt. Die Essigester-Phase wird noch einmal mit Wasser ausgeschüttelt. Die vereinigten Wasserphasen werden mit einem schwach basischen Ionenaustauscher in Acetat-Form verrührt, bis sich ein pH von 3-4 einstellt. Der Ionenaustauscher wird abfiltriert und das Filtrat gefriergetrocknet.

Ausbeute 65 mg.

Aminosäureanalyse: Cys 1,83; Phe 2,0; Lys 1,0; Trp 1,0 (wurde über das UV-Spektrum ermittelt); Gehalt an Peptidbase laut Aminosäureanalyse: 55 %.

### 16. H-Arg-Tyr(tBu)-OtBu

### 16a. Z-Arg(Z$_2$)-Tyr(tBu)-OtBu, C$_{47}$H$_{57}$N$_5$O$_{10}$ (MG 852,0)

Zu einer Lösung von 2,89 g (5 mmol) Z-Arg-(Z$_2$)-OH, 1,65 g H-Tyr(tBu)-OtBu•HCl, 0,68 g HOBt und 0,65 ml N-Ethylmorpholin gibt man unter Rühren bei 0°C 1,1 g DCC. Der Ansatz wird analog Beispiel 1a aufgearbeitet. Der Rückstand wird zur Reinigung an Kieselgel in Methylenchlorid/Methanol wie 95:5 chromatographiert. Die einheitlichen Fraktionen werden vereinigt und im Vakuum eingeengt. Der Rückstand wird mit Petrolether verrieben.
Ausbeute 2,57 g, Schmp. 123-124°C, $[\alpha]_D^{25}$ = +9,6° (c = 1, in Eisessig).

### 16b. H-Arg-Tyr(tBu)-OtBu•2HCl, C$_{23}$H$_{41}$Cl$_2$N$_5$O$_4$ (MG 522,5)

2,04 g Z-Arg(Z$_2$)-Tyr(tBu)-OtBu werden analog Beispiel 2a katalytisch hydriert. Der Rückstand wird mit Diethylether verrieben.
Ausbeute 1,06 g, amorph, $[\alpha]_D^{22}$ = +22,6° (c = 1, Methanol).

### 17. Fmoc-Cys(StBu)-Arg-Phe-OtBu

### 17a. Z-Arg(Z$_2$)-Phe-OtBu, C$_{43}$H$_{49}$N$_5$O$_9$ (MG 779,9)

Zu einer Lösung von 17,3 g (30 mmol) Z-Arg(Z$_2$)-OH, 7,73 g H-Phe-OtBu•HCl, 4,9 g HOObt und 3,9 ml N-Ethylmorpholin in 70 ml Dimethylformamid gibt man unter Rühren bei 0°C 6,6 g DCC. Man arbeitet analog Beispiel 1a auf. Der Rückstand wird mit Diethylether verrieben, abgesaugt und im Vakuum getrocknet.
Ausbeute 19 g, $[\alpha]_D^{20}$ = -0,5° (c = 1, in 80 %iger wäßriger Essigsäure).

### 17b. H-Arg-Phe-OtBu•2HClO$_4$, C$_{19}$H$_{33}$Cl$_2$N$_5$O$_{11}$ (MG 578,4)

18,6 g Z-Arg(Z$_2$)-Phe-OtBu werden in 200 ml Dimethylacetamid gelöst und über Pd/C katalytisch mit Wasserstoff hydriert. Mit Hilfe eines Autotitrators wird während der Hydrierung durch Zusatz von 1N HClO$_4$ pH 5 gehalten. Nach beendigter Hydrierung wird der Katalysator abgesaugt und das Filtrat eingeengt. Der Rückstand wird in Wasser gelöst, über eine Klärschicht filtriert und gefriergetrocknet.
Ausbeute 21,1 g Öl.
Das Öl wird zweimal mit Diethylether verrieben, der Diethylether abdekantiert und der ölige Rückstand im Vakuum getrocknet.
Ausbeute 18,5 g (135 %, enthält also noch Lösungsmittel).

### 17c. Fmoc-Cys(StBu)-Arg-Phe-OtBu-acetat, C$_{43}$H$_{58}$N$_6$O$_8$S$_2$ (MG 851,1)

Zu einer Lösung von 4,31 g (10 mmol) Fmoc-Cys(StBu)-OH, 7,74 g H-Arg-Phe-OtBu•2HClO$_4$ (oben gewonnene ölige Substanz), 1,35 g HOBt und 1,3 ml N-Ethylmorpholin in 40 ml Dimethylformamid gibt man bei 0°C unter Rühren 2,2 g DCC. Der Ansatz wird analog Beispiel 1a aufgearbeitet. Der Rückstand wird mit Diethylether verrieben und getrocknet.
Ausbeute 8 g.

1,3 g der oben gewonnenen Substanz werden in 30 ml 55 %iger Essigsäure gelöst und über einen - schwach basischen Ionenaustauscher in Acetat-form chromatographiert. Man eluiert mit 55 %iger Essigsäure. Das Eluat wird eingeengt, in Wasser aufgenommen und gefriergetrocknet.

Ausbeute 1,05 g, $[\alpha]_D^{29}$ = -45,5° (c = 1, in Methanol), Gehalt an Peptidbase laut Aminosäureanalyse: 79 %.

## 18. Fmoc-Arg-Pro-Cys(StBu)-Arg-Phe-OtBu

### 18a. Fmoc-Arg-Pro-OtBu, $C_{30}H_{39}N_5O_5$ (MG 549,68)

Zu einer Lösung von 5,95 g (15 mmol) Fmoc-Arg-OH, 2,57 g H-Pro-OtBu, 2,02 g HOBt und 2,7 g Pyridiniumperchlorat in 30 ml Dimethylformamid gibt man bei 0°C unter Rühren 3,3 g DCC. Man arbeitet analog Beispiel 1a auf. Der Rückstand wird mit Diethylether verrieben und abgesaugt.
Ausbeute 9,4 g.

Zur Charakterisierung werden 750 mg der oben gewonnenen Substanz in 40 %iger wäßriger Essigsäure gelöst und über einen schwach basischen Ionenaustauscher in Acetat-Form chromatographiert. Das Eluat wird eingeengt und gefriergetrocknet.

Ausbeute 690 mg Fmoc-Arg-Pro-OtBu-Acetat, $C_{32}H_{43}N_5O_7$ (MG 609,7), $[\alpha]_D^{20}$ = -44,2° (c = 1, in Methanol), Gehalt an Substanz laut Aminosäureanalyse: 93 %.

### 18b. Fmoc-Arg-Pro-OH, $C_{26}H_{31}N_5O_5$ (MG 493,57)

8,5 g oben gewonnener Rohsubstanz wird in 85 ml 90 %iger wäßriger Trifluoressigsäure gelöst. Man läßt 1 Stunde bei Raumtemperatur stehen und engt im Vakuum ein. Der Rückstand wird mit Diethylether verrieben und abgesaugt.
Ausbeute 7,9 g.

7,16 g der oben gewonnenen Substanz werden in 100 ml Wasser suspendiert und mit einer gesättigten $NaHCO_3$-Lösung ein pH-Wert von 6 eingestellt. Man stellt übers Wochenende bei 4°C und saugt den Niederschlag ab und trocknet über $P_2O_5$ im Vakuum.
Ausbeute 6,15 g, $[\alpha]_D^{20}$ = -32,2° (c = 1, in 80 %iger wäßriger Essigsäure.

### 18c. H-Cys(StBu)-Arg-Phe-OtBu, $C_{26}H_{44}N_6O_4S_2$ (MG 568,8)

Zu einer Lösung von 17,84 g (20 mmol) Fmoc-Cys(StBu)-Arg-Phe-OtBu•HClO₄ (hergestellt analog Rohsubstanz von Beispiel 17c) in 100 ml Dimethylformamid gibt man bei Raumtemperatur 21 ml (200 mmol) Diethylamin. Man rührt 10 Minuten bei Raumtemperatur und engt im Vakuum ein. Der Rückstand wird zweimal mit Diethylether verrieben und dekantiert. Der ölige Rückstand wird im Hochvakuum getrocknet.
Ausbeute 14,6 g.

### 18d. Fmoc-Arg-Pro-Cys(StBu)-Arg-Phe-OtBu-diacetat, $C_{56}H_{81}N_{11}O_{12}S_2$ (MG 1164,46)

Zu einer Lösung von 4,94 g (10 mmol) Fmoc-Arg-Pro-OH, 5,7 g (10 mmol) H-Cys(StBu)-Arg-Phe-OtBu, 163 mg HOObt und 1,8 g Pyridinperchlorat in 100 ml Dimethylformamid gibt man bei 0°C 2,2 g DCC. Man arbeitet analog Beispiel 1a auf. Der Rückstand wird mit Diethylether verrieben.
Ausbeute 8,7 g.

500 mg der oben gewonnenen Substanz werden an Kieselgel chromatographiert. Als Elutionsmittel dient folgende Mischung: 450 ml Methylenchlorid, 200 ml Methanol, 150 g Essigsäure bis zu einem pH von 6,8.
Ausbeute 341 mg, $[\alpha]_D^{22}$ = -60,8° (c = 1, Wasser), Gehalt an Peptidbase laut Aminosäureanalyse: 77 %/

### 19. H-Arg-Pro-Cys(StBu)-Arg-Phe-OtBu-triacetat, $C_{43}H_{75}N_{11}O_{12}S_2$ (MG 1002,27)

Zu einer Lösung von 3,3 g Fmoc-Arg-Pro-Cys(StBu)-Arg-Phe-OtBu in 20 ml Dimethylformamid gibt man 3,32 ml Diethylamin. Man läßt 10 Minuten bei Raumtemperatur stehen und engt im Vakuum ein. Der Rückstand wird mit Diethylether verrieben und abgesaugt.
Ausbeute 2,7 g.
Die oben gewonnene Substanz wird analog Beispiel 18d chromatographisch gereinigt.
Ausbeute 1,23 g, $[\alpha]_D$ = -42,4° (c = 1 in Wasser), Gehalt an Peptidbase laut Aminosäureanalyse: 71 %.

### 20. Z-Trp-Asp(OtBu)-Arg-Phe-NH$_2$

### 20a. Z-Arg(Z$_2$)-Phe-NH$_2$, $C_{39}H_{42}N_6O_8$ (MG 722,8)

Zu einer Lösung von 8,65 g (15 mmol) Z-Arg(Z$_2$)-OH, 3,01 g H-Phe-NH$_2$·HCl, 2,45 g HOObt und 1,95 ml N-Ethylmorpholin in 30 ml Dimethylformamid gibt man bei 0°C unter Rühren 3,3 g DCC. Man verfährt weiter wie bei Beispiel 1a. Am anderen Tag war der Ansatz erstarrt. Man vereibt nun mit halbgesättigter NaHCO$_3$-Lösung, saugt ab, verrührt mit KHSO$_4$/K$_2$SO$_4$-Lösung, saugt ab, wäscht mit Wasser und trocknet über P$_2$O$_5$. Die Substanz enthält in etwa equimolaren Mengen den Dicyclohexylharnstoff.
Ausbeute 13,9 g.

### 20b. H-Arg-Phe-NH$_2$·2HClO$_4$, $C_{15}H_{26}Cl_2N_6O_{10}$ (MG 521,33)

13,5 g der oben gewonnenen Substanz (Z-Arg(Z$_2$)-Phe-NH$_2$·Dicyclohexylharnstoff) werden in 200 ml Dimethylacetamid verrührt und mit Wasserstoff und Pd/C katalytisch hydriert. Mit Hilfe eines Autotitrators wird durch Zugabe von 1N HClO$_4$ ein pH von 5 eingehalten. Nach beendigter Hydrierung saugt man den Niederschlag ab und engt das Filtrat im Vakuum ein. Der Rückstand wird mit Wasser verrührt und von Unlöslichem wird abgesaugt. Das Filtrat wird gefriergetrocknet. Der ölige Rückstand wird zweimal mit Diethylether verrieben und dekantiert. Das Öl wird im Hochvakuum getrocknet.
Ausbeute 9,5 g. Das Öl enthält noch Lösungsmittel.

### 20c. Z-Asp(OtBu)-Arg-Phe-NH$_2$·HClO$_4$, $C_{31}H_{44}Cl_1N_7O_{11}$ (MG 726,2)

Zu einer Lösung von 5,9 g Z-Asp(OtBu)-OH, 9,5 g H-Arg-Phe-NH$_2$·2HClO$_4$, 2,48 g HOBt und 2,37 ml N-Ethylmorpholin in 50 ml Dimethylformamid gibt man bei 0°C unter Rühren 4 g DCC. Der Ansatz wird analog Beispiel 1a aufgearbeitet. Der Rückstand wird mit Diethylether verrieben und abgesaugt.
Ausbeute 9 g.

### 20d. H-Asp(OtBu)-Arg-Phe-NH$_2$·2HClO$_4$, $C_{23}H_{39}Cl_2N_7O_{13}$ (MG 692,5)

8,7 g Z-Asp(OtBu)-Arg-Phe-NH$_2$·HClO$_4$, werden in 150 ml Methanol gelöst und analog Beispiel 20b hydriert. Der Rückstand wird mit Diethylether verrieben und abgesaugt.
Ausbeute 7,8 g, $[\alpha]_D^{20}$ = -4,0° (c = 1, in Methanol).

22

### 20e. Z-Trp-Asp(OtBu)-Arg-Phe-NH$_2$-acetat, C$_{44}$H$_{57}$N$_9$O$_{10}$ (MG 872)

Zu einer Lösung von 3,4 g Z-Trp-OH (10 mmol), 6,92 g H-Asp(OtBu)-Arg-Phe-NH$_2$·2HClO$_4$, 1,35 g HOBt und 1,3 ml N-Ethylmorpholin in 40 ml Dimethylformamid gibt man bei 0°C unter Rühren 2,2 g DCC. Man arbeitet analog Beispiel 1a auf. Der Rückstand wird mit Diethylether verrieben und abgesaugt. Ausbeute 7,3 g.

220 mg der oben gewonnenen Substanz werden in 15 ml 55 %iger wäßriger Essigsäure gelöst und über einen schwach basischen Ionenaustauscher in Acetatform chromatographiert. Man eluiert mit 55 %iger Essigsäure. Das Eluat wird eingeengt und der Rückstand in Wasser aufgenommen und gefriergetrocknet. Ausbeute 180 mg, $[\alpha]_D^{29}$ = -19,2° (c = 1, in Methanol), Gehalt an Peptidbase laut Aminosäureanalyse: 75%.

### 21. H-Arg-Trp-Asp-(OtBu)-Arg-Phe-NH$_2$

### 21a. Z-Arg(Z$_2$)-Trp-Asp(OtBu)-Arg-Phe-NH$_2$, C$_{64}$H$_{79}$N$_{13}$O$_{13}$ (MG 1238,4)

Zu einer Lösung von 576 mg (1 mmol) Z-Arg(Z$_2$)-OH, 879 mg H-Trp-Asp(OtBu)-Arg-Phe-NH$_2$·HClO$_4$, 163 mg HOObt und 0,13 ml N-Ethylmorpholin in 10 ml Dimethylformamid gibt man unter Rühren bei 0°C 220 mg DCC. Man läßt über Nacht bei Raumtemperatur stehen, saugt den Niederschlag ab und engt das Filtrat im Hochvakuum ein. Der Rückstand wird zwischen n-Pentanol und gesättigter NaHCO$_3$-Lösung verteilt. Dabei bleibt das Meiste ungelöst und wird abgesaugt. Ausbeute 910 mg.

Die n-Pentanolphase wird eingeengt. Ausbeute 250 mg, $[\alpha]_D^{20}$ = -15,2° (c = 1, in 80 %iger wäßriger Essigsäure).

### 21b. H-Arg-Trp-Asp(OtBu)-Arg-Phe-NH$_2$-triacetat, C$_{46}$H$_{71}$N$_{13}$O$_{13}$ (MG 1014,15)

3 g Z-Arg(Z$_2$)-Trp-Asp(OtBu)-Arg-Phe-NH$_2$ werden in 50 ml 90 %iger wäßriger Essigsäure gelöst und über Pd/C mit Wasserstoff katalytisch hydriert. Nach beendigter Hydrierung wird das Katalysator abgesaugt und das Filtrat eingeengt. Der Rückstand wird mit Diethylether verrieben und abgesaugt. Ausbeute 2,33 g.

960 mg der oben gewonnenen Substanz werden analog Beispiel 18d chromatographisch gereinigt. Ausbeute 540 mg, $[\alpha]_D^{22}$ = -14,2° (c = 1, in Wasser), Gehalt an Peptidbase laut Aminosäureanalyse: 84 %.

### 22. Z-Asp(OtBu)-Arg-Trp-NH$_2$

### 22a. Z-Arg-(Z$_2$)-Trp-NH$_2$, C$_{41}$H$_{43}$N$_7$O$_8$ (MG 761,85)

Zu einer Lösung von 17,3 g (30 mmol) Z-Arg(Z$_2$)-OH, 7,2 g H-Trp-NH$_2$·HCl, 4,9 g HOObt und 3,9 ml N-Ethylmorpholin in 150 ml Dimethylformamid gibt man bei 0°C unter Rühren 6,6 g DCC. Man arbeitet analog Beispiel 7a auf. Ausbeute 20,5 g.

Zur Reinigung verrührt man die Substanz mit Methanol, saugt ab, wäscht mit Diethylether und trocknet im Vakuum. Ausbeute 20,3 g, Schmp. 172-174°C, $[\alpha]_D^{22}$ = -1,4° (c = 1, in Dimethylformamid).

**22b. H-Arg-Trp-NH$_2$ •2 p-Toluolsulfonsäure, C$_{31}$H$_{41}$N$_7$O$_8$S$_2$ (MG 703,8)**

17,2 g Z-Arg(Z$_2$)-Trp-NH$_2$ werden in 350 ml Methanol suspendiert und analog Beispiel 2a katalytisch hydriert (statt methanolischer HCl wird eine 1N-p-Toluolsulfonsäure-Lösung in Methanol verwendet). Der Rückstand wird mit Diethylether verrieben und abgesaugt.
Ausbeute 12,3 g, $[\alpha]_D^{22} = +13,3°$ (c = 1, in Methanol).

**22c. Z-Asp(OtBu)-Arg-Trp-NH$_2$-acetat, C$_{35}$H$_{48}$N$_8$O$_9$ (MG 724,8)**

Zu einer Lösung von 13,5 g H-Arg-Trp-NH$_2$ •2 p-Toluolsulfonsäure und 2,49 ml N-Ethylmorpholin gibt man bei Raumtemperatur unter Rühren 8,06 g Z-Asp(OtBu)-ONSu. Man läßt über Nacht bei Raumtemperatur stehen. Man engt ein und verteilt den Rückstand zwischen n-Pentanol und Wasser. Die organische Phase wird eingeengt und der Rückstand mit Diethylether verrieben und abgesaugt.
Ausbeute 14,4 g Z-Asp(OtBu)-Arg-Trp-NH$_2$ •p-Toluolsulfonsäure, $[\alpha]_D^{23} = -16,4°$ (c = 1, in Methanol).
850 mg der oben gewonnenen Substanz werden in 15 ml 30 %iger wäßriger Essigsäure gelöst und über einen schwach basischen Ionenaustauscher in Acetatform chromatographiert. Eluiert wird mit 30 %iger Essigsäure. Das Eluat wird eingeengt und der Rückstand in Wasser gelöst. Die Lösung wird gefriergetrocknet.
Ausbeute 750 mg, $[\alpha]_D^{29} = -16,6°$ (c = 1, in Methanol), Gehalt an Peptidbase laut Aminosäureanalyse 85 %.

**23. Z-Arg-Ile-Asp(OtBu)-Arg-Trp-NH$_2$**

**23a. H-Asp(OtBu)-Arg-Trp-NH$_2$ •p-Toluolsulfonsäure, C$_{39}$H$_{54}$N$_8$O$_{11}$S$_2$ (MG 875,05)**

13,2 g Z-Asp(OtBu)-Arg-Trp-NH$_2$ •p-Toluolsulfonsäure werden analog Beispiel 22b katalytisch hydriert.
Ausbeute 13,04 g, $[\alpha]_D^{22} = -3,1°$ (c = 1, in Methanol).

**23b. Z-Arg-Ile-Asp(OtBu)-Arg-Trp-NH$_2$-diacetat, C$_{49}$H$_{75}$N$_{13}$O$_{13}$ (MG 1054,2)**

Zu einer Lösung von 843 mg (2 mmol) Z-Arg-Ile-OH, 1,75 g H-Asp(OtBu)-Arg-Trp-NH$_2$ •2 p-Toluolsulfonsäure und 326 mg HOObt in 20 ml Diethylformamid gibt man bei 0°C unter Rühren 440 mg DCC. Man läßt über Nacht bei Raumtemperatur stehen, saugt den Niederschlag ab und engt das Filtrat ein. Den Rückstand unterwirft man einer 5-stufigen Gegenstromverteilung zwischen Essigester und Wasser. Das Peptid findet sich in den Wasserphasen, die gefriergetrocknet werden.
Ausbeute 2,01 g Z-Arg-Ile-Asp(OtBu)-Arg-Trp-NH$_2$ •2 p-Toluolsulfonsäure, $[\alpha]_D^{20} = -25°$ (c = 1, in Methanol).
150 mg des oben gewonnenen Peptids werden analog Beispiel 22c in das Acetat überführt.
Ausbeute 120 mg, $[\alpha]_D^{19} = -30,8°$ (c = 1, in Methanol), Gehalt an Peptidbase laut Aminosäureanalyse: 73 %.

**24. H-Arg-Ile-Asp(OtBu)-Arg-Trp-NH$_2$-triacetat, C$_{43}$H$_{73}$N$_{13}$O$_{13}$ (MG 980,1)**

1,73 g Z-Arg-Ile-Asp(OtBu)-Arg-Trp-NH$_2$ •2 p-Toluolsulfonsäure werden in 90 %iger wäßriger Essigsäure über Pd/C katalytisch hydriert. Der Katalysator wird abgesaugt, das Filtrat eingeengt und der Rückstand mit Diethylether verrieben und abgesaugt. Die Substanz wird in Wasser gelöst und mit einem schwach basischen Ionenaustauscher in Acetatform gerührt bis sich ein pH von 4,4 eingestellt hat. Der Austauscher wird abfiltriert und das Filtrat gefriergetrocknet.
Ausbeute 1,06 g.
Die oben gewonnene Substanz wird analog Beispiel 18d chromatographisch gereinigt.
Ausbeute 634 mg, $[\alpha]_D^{23} = -31,9°$ (c = 1, in Wasser). Gehalt an Peptidbase laut UV-Spektrum: 72 %.

## 25. Z-D-Phe-Trp-Lys-Tyr-Phe-OBzl

### 25a. Fmoc-Tyr(tBu)-Phe-OBzl

Zu einer Lösung von 9,18 g (20 mmol) Fmoc-Tyr(tBu)-OH, 8,5 g (20 mmol) H-Phe-OBzl•p-Toluolsulfonsäure, 2,8 g HOBt und 2,56 ml N-Ethylmorpholin in 50 ml Dimethylformamid gibt man bei 0°C unter Rühren 4,74 g DCC. Man arbeitet analog Beispiel 1a auf. Aus Essigester/Petrolether wird kristallisiert. Ausbeute 11,4 g (85 %), Schmp. 158°C unter Zersetzung, $[\alpha]_D^{21}$ = -34,5° (c = 1, in Dimethylformamid)

### 25b. H-Tyr(tBu)-Phe-OBzl

Zu einer Lösung von 10,44 g (15 mmol) Fmoc-Tyr(tBu)-Phe-OBzl in 100 ml Dimethylformamid gibt man 15,64 ml (150 mmol) Diethylamin. Man läßt 10 Minuten bei Raumtemperatur rühren, engt ein und verreibt den Rückstand mit Petrolether. Der Petrolether wird abdekantiert und der Rückstand in Diethylether gelöst. Man filtriert von wenig Ungelöstem und engt das Filtrat ein. Der Rückstand wird im Hochvakuum getrocknet.
Ausbeute 5,2 g.

### 25c. Fmoc-Lys(Boc)-Tyr(tBu)-Phe-OBzl, $C_{55}H_{64}N_4O_9$ (MG 925,15)

Zu einer Lösung von 4,38 g Fmoc-Lys(Boc)-OH, 5,2 g H-Tyr(tBu)-Phe-OBzl und 1,31 g HOObt in 50 ml Dimethylformamid gibt man bei 0°C unter Rühren 1,97 g DCC. Man arbeitet analog Beispiel 1a auf. Kristallisation aus Essigester/Petrolether.
Ausbeute 6,5 g.

### 25d. H-Lys(Boc)-Tyr(tBu)-Phe-OBzl, $C_{40}H_{54}N_4O_7$ (MG 702,9)

Zu einer Lösung von 6,5 g (7 mmol) Fmoc-Lys(Boc)-Tyr(tBu)-Phe-OBzl in 50 ml Dimethylformamid gibt man 7,3 ml Diethylamin und rührt 10 Minuten bei Raumtemperatur. Danach wird im Vakuum eingeengt und der Rückstand mit Diethylether verrieben.
Ausbeute 5,53 g, Schmp. 132°C, $[\alpha]_D^{21}$ = 0°. (c = 1, in Dimethylformamid.

### 25e. Z-D-Phe-Trp-OMe, $C_{29}H_{29}N_3O_5$ (MG 499,5)

Zu einer Lösung von 7,47 g (25 mmol) Z-D-Phe-OH, 6,37 g (25 mmol) H-Trp-OMe•HCl, 3,37 g HOBt und 3,2 ml N-Ethylmorpholin in 50 ml Dimethylformamid gibt man bei 0°C unter Rühren 5,56 g DCC. Man arbeitet analog Beispiel 1a auf. Die Substanz kristallisiert aus Essigester.
Ausbeute 8,13 g, Schmp. 181°C, $[\alpha]_D^{23}$ = +2,5° (c = 1, in Dimethylformamid).

### 25f. Z-D-Phe-Trp-OH, $C_{28}H_{27}N_3O_5$ (MG 485,5)

Zu einer Lösung von 2,9 g Z-D-Phe-Trp-OMe in 20 ml Dioxan/Wasser (4:1) gibt man 6,1 ml 1N NaOH. Man läßt 2 Stunden bei Raumtemperatur rühren und versetzt dann mit 6,1 ml 1N HCl. Die Lösung wird eingeengt und der Rückstand mit Wasser verrieben. Der Niederschlag wird abgesaugt und über $P_2O_5$ getrocknet.
Ausbeute 2,27 g, Schmp. 143-145°C, $[\alpha]_D^{21}$ = +4,4° (c = 1, in Dimethylformamid).

### 25g. Z-D-Phe-Trp-Lys(Boc)-Tyr(tBu)-Phe-OBzl, $C_{68}H_{72}N_7O_{11}$ (MG 1170,4)

Zu einer Lösung von 2,4 g (5 mmol) Z-D-Phe-Trp-OH, 3,1 g H-Lys(Boc)-Tyr(tBu)-Phe-OBzl und 0,7 g HOBt in 30 ml Dimethylformamid gibt man bei 0°C unter Rühren 1,13 g DCC. Man arbeitet analog Beispiel 1a auf. Aus Diethylether/Petrolether wird kristallisiert.
Ausbeute 5,62 g (96 %), Schmp. 194°C unter Zersetzung, $[\alpha]_D^{21}$ = -8,5° (c = 1, in Dimethylformamid).

### 25h. Z-D-Phe-Trp-Lys-Tyr-Phe-OBzl-acetat, $C_{61}H_{67}N_7O_{11}$ (MG 1074,2)

1,1 g (1 mmol) Z-D-Phe-Trp-Lys(Boc)-Tyr(tBu)-Phe-OBzl werden analog Beispiel 2c umgesetzt. Der Rückstand wird mit Methyl-tert.-butyl-ether verrieben und abgesaugt. Die Substanz wird mit halbgesättigter $NaHCO_3$-Lösung verrührt und abgesaugt. Die Substanz wird in 20 ml Methanol gelöst und mit 20 ml Wasser gefällt.
Ausbeute 0,41 g.
Zur Überführung in das Acetat wird oben gewonnene Substanz in 3 ml Eisessig gelöst. Man versetzt mit 70 ml Wasser und gefriertrocknet.
Ausbeute 0,4 g.
Aminosäureanalyse: Tyr 0,66; Phe 1,97; Lys 1,0; Gehalt an Peptidbase laut Aminosäureanalyse: 75 %.

### 26. Cyclo-(D-Phe-Trp-Lys-Tyr-Phe)

### 26a. H-D-Phe-Trp-Lys(Boc)-Tyr(tBu)-Phe-OH, $C_{53}H_{67}N_7O_9$ (MG 946,2)

4,45 g (3,8 mmol) Z-D-Phe-Lys(Boc)-Tyr(tBu)-Phe-OBzl werden in 100 ml 90 %iger Essigsäure über Pd/C katalytisch hydriert. Nach beendigter Hydrierung wird der Katalysator abgesaugt und das Filtrat eingeengt. Der Rückstand wird mit Diethylether verrieben, abgesaugt und getrocknet.
Ausbeute 3,25 g (90,5 %).
Die Substanz wird zur Entfernung von anhaftender Essigsäure mit soviel 10 %iger $NaHCO_3$-Lösung verrührt bis sich ein pH von 6 einstellt. Danach wird die Substanz abgesaugt, mit Wasser gewaschen und über $P_2O_5$ getrocknet.
Ausbeute 2,81 g, Schmp. 225-227°C unter Zersetzung, $[\alpha]_D^{21}$ = -22,7° (c = 1, in Dimethylformamid).

### 26b. Cyclo-(D-Phe-Trp-Lys(Boc)-Tyr(tBu)-Phe), $C_{53}H_{65}N_7O_8$ (MG 928,2)

Zu einer Lösung von 0,946 g (1 mmol) H-D-Phe-Trp-Lys(Boc)-Tyr(tBu)-Phe-OH in 125 ml Dimethylformamid gibt man 0,35 ml (2,5 mmol) einer 50 %igen Lösung von Ethyl-methylphosphinsäure-anhydrid in Methylenchlorid. Innerhalb von 30 Minuten läßt man bei Raumtemperatur langsam 0,64 ml (5 mmol) N-Ethylmorpholin, gelöst in 25 ml Dimethylformamid, zutropfen. Man rührt 3 Stunden bei Raumtemperatur nach und engt dann den Ansatz ein. Der Rückstand wird mit Wasser verrieben, abgesaugt und getrocknet.
Ausbeute 0,79 g.
Zur Abtrennung von nicht cyclisiertem Material wird in 20 ml Methanol und 20 ml Wasser heiß gelöst. Nach dem Abkühlen wird abgesaugt.
Ausbeute 0,33 g.
Zur weiteren Reinigung wird die Substanz in Methylenchlorid/Methanol wie 95:5 an Kieselgel chromatographiert.
Ausbeute 131 mg.

### 26c. Cyclo-(D-Phe-Trp-Lys-Tyr-Phe)-acetat, $C_{46}U_{53}N_7O_8$ (MG 832)

105 mg Cyclo-(D-Phe-Trp-Lys(Boc)-Tyr(tBu)-Phe) werden analog Beispiel 2c behandelt. Es wird analog Beispiel 25h aufgearbeitet.
Ausbeute 50,3 mg.
Aminosäureanalyse: Tyr 0,89; Phe 2,03; Lys 1,0; Gehalt an Peptidbase laut Aminosäureanalyse: 76 %.

### 27. Z-Lys-Lys-Tyr-Phe-OMe

### 27a. H-Lys(Boc)-Tyr(tBu)-Phe-OMe·HCl

28 g Z-Lys(Boc)-Tyr(tBu)-Phe-OMe (siehe Beispiel 4c) werden analog Beispiel 2a in Methanol katalytisch hydriert.
Ausbeute 22,4 g (91,8 %), Schmp. 145-147°C, $[\alpha]_D^{24} = +18,2°$ (c = 1, in Methanol).

### 27b. Z-Lys(Boc)-Lys(Boc)-Tyr(tBu)-Phe-OMe

Zu einer Lösung von 21,9 g H-Lys(Boc)-Tyr(tBu)-Phe-OMe·HCl, 4,46 g HOBt und 4,3 ml N-Ethylmorpholin in 130 ml Dimethylformamid gibt man bei Raumtemperatur 18,5 g Z-Lys(Boc)-OTcp und rührt 3 Stunden bei Raumtemperatur. Man versetzt den Ansatz mit 400 ml Wasser und 35 ml gesättigter NaHCO₃-Lösung. Der Niederschlag wird abgesaugt. Aus 200 ml Essigester und 800 ml Petrolether wird umgefällt.
Ausbeute 30,4 g (93 %), Schmp. 159-160°C, $[\alpha]_D^{28} = -17,5°$ (c = 1, in Methanol).

### 27c. Z-Lys-Lys-Tyr-Phe-OMe-acetat

1,0 g Z-Lys(Boc)-Lys(Boc)-Tyr(tBu)-Phe-OMe werden in 10 ml 90 %iger Trifluoressigsäure gelöst. Man läßt 1 Stunde bei Raumtemperatur stehen. Danach wird eingeengt und der Rückstand mit Diethylether verrieben und abgesaugt. Der Rückstand wird in Wasser gelöst und über einen schwach basischen Ionenaustauscher in Acetatform chromatographiert. Das Eluat wird gefriergetrocknet. Ausbeute 736,8 mg. Nach chromatographischer Reinigung an Kieselgel:
Ausbeute 576 mg, $[\alpha]_D^{21} = -31,1°$ (c = 1, in Wasser).

### 28. Fmoc-Arg-Tyr(tBu)-Phe-NH₂-acetat, $C_{45}H_{55}N_7O_8$ (MG 822)

Zu einer Lösung von 3,96 g (10 mmol) Fmoc-Arg-OH, 4,2 g (10 mmol) H-Tyr(tBu)-Phe-NH₂·HCl (siehe Beispiel 7b) und 1,4 g (10 mmol) HOBt in 50 ml Dimethylformamid gibt man bei 0° 2,2 g DCC. Man läßt über Nacht bei Raumtemperatur rühren. Danach wird eingeengt und der Rückstand mit gesättigter NaHCO₃-Lösung verrieben. Der Niederschlag wird abgesaugt und aus Methanol/Wasser umgefällt. Ausbeute 6,1 g. Zur Reinigung wird die Substanz an Kieselgel chromatographiert (Laufmittel: Methylenchlorid/Methanol/Wasser/Eisessig wie 9:1:0,1:0,1.
Ausbeute 3,85 g, $[\alpha]_D^{22} = -7,1°$ (c = 1, in 90 %iger Essigsäure).

### 29. Fmoc-D-Arg-Tyr(tBu)-Phe-NH₂-acetat, $C_{45}H_{55}N_7O_8$ (MG 822)

Wird analog Beispiel 28 hergestellt.
$[\alpha]_D^{22} = +8,7°$ (c = 1, in 90 %iger Essigsäure).

### 30. Z-Trp-Arg-Tyr-Phe-NH$_2$

### 30a. H-Arg-Tyr(tBu)-Phe-NH$_2$-acetat, C$_{30}$H$_{45}$N$_7$O$_6$ (MG 599,7)

Zu einer Lösung von 3,05 g Fmoc-Arg-Tyr(tBu)-Phe-NH$_2$-acetat in 25 ml Dimethylformamid gibt man 4,2 ml Diethylamin. Man läßt 5 Minuten bei Raumtemperatur stehen und engt im Vakuum ein. Der Rückstand wird mit Diethylether verrieben und abgesaugt.
Ausbeute 2,23 g.

### 30b. Z-Trp-Arg-Tyr(tBu)-Phe-NH$_2$-acetat, C$_{49}$H$_{61}$N$_9$O$_9$ (MG 920,1)

Zu einer Lösung von 2,15 g (3,59 mmol), 0,56 g HOBt und 0,64 g (3,59 mmol) Pyridinium-perchlorat in 25 ml Dimethylformamid gibt man bei Raumtemperatur 2,17 g Z-Trp-OTcp. Man läßt über Nacht bei Raumtemperatur stehen und engt anderntags ein. Man verteilt den Rückstand zwischen Essigester und gesättigter NaHCO$_3$-Lösung. Die Essigester-Phase wird über Na$_2$SO$_4$ getrocknet und eingeengt. Der Rückstand wird mit Diethylether verrieben. Ausbeute 2,61 g. Zur Reinigung wird an Kieselgel wie bei Versuch 28a chromatographiert.
Ausbeute 1 g.

### 30c. Z-Trp-Arg-Tyr-Phe-NH$_2$-acetat, C$_{45}$H$_{53}$N$_9$O$_9$ (MG 864)

700 mg Z-Trp-Arg-Tyr(tBu)-Phe-NH$_2$ werden analog Beispiel 2c behandelt.
Ausbeute 0,38 g, $[\alpha]_D^{22}$ = -5,7° (c = 1, in 90 %iger Essigsäure).

### 31. Z-Trp-D-Arg-Tyr-Phe-NH$_2$-acetat, C$_{45}$H$_{53}$N$_3$O$_3$ (MG 864)

Wird analog Beispiel 30 hergestellt.
$[\alpha]_D^{22}$ = +6,0° (c = 1, in 90 %iger Essigsäure).

### 32. Z-Trp-D-Lys-Tyr-Phe-NH$_2$, C$_{43}$H$_{49}$N$_7$O$_7$ (MG 775,9)

Wird analog Beispiel 8 hergestellt.
$[\alpha]_D^{22}$ = +6,5° (c = 1, in 90 %iger Essigsäure)

### 33. Indol-3-butyryl-Trp-Lys-Tyr-Phe-NH$_2$

### 33a. Indol-3-butyryl-Trp-Lys(Boc)-Tyr(tBu)-Phe-NH$_2$, C$_{56}$H$_{70}$N$_8$O$_8$ (MG 983,24)

Zu einer Lösung von 509 mg Indol-3-buttersäure, 2,1 g H-Trp-Lys(Boc)-Tyr(tBu)-Phe-NH$_2$·HCl (siehe Beispiel 8c, katalytische Hydrierung analog Beispiel 2a), 337 mg HOBt und 0,325 ml N-Ethylmorpholin in 20 ml Dimethylformamid gibt man bei 0°C 550 mg DCC. Aufarbeitung wie bei Beispiel 1a. Der Rückstand wird mit Diethylether verrieben und abgesaugt.
Ausbeute 2,15 g, Schmp. 148-155°C, $[\alpha]_D^{23}$ = -15,4° (c = 1, in Methanol).

### 33b. Indol-3-butyryl-Trp-Lys-Tyr-Phe-NH$_2$-acetat, C$_{49}$H$_{58}$N$_8$O$_8$ (MG 887,05)

1,97 g Indol-3-butyryl-Trp-Lys(Boc)-Tyr(tBu)-Phe-NH$_2$ werden analog Beispiel 2c umgesetzt. Ausbeute 1,29 g. Gereinigt wird durch Säulenchromatographie an Kieselgel mit Methylenchlorid/Methanol/Wasser/Eisessig wie 7:2:0,3:0,15.
Ausbeute 397 mg, $[\alpha]_D^{23}$ = -2,7° (c = 1, in 10 %iger Essigsäure).

### 34. Indol-3-butyryl-Lys-Tyr-Phe-NH$_2$

### 34a. Indol-3-butyryl-Lys(Boc)-Tyr(tBu)-Phe-NH$_2$, C$_{45}$H$_{60}$N$_6$O$_7$ (MG 797,02)

Zu einer Lösung von 1,95 g (3 mmol) H-Lys(Boc)-Tyr(tBu)-Phe-NH$_2$•HCl, 610 mg Indol-3-buttersäure, 405 mg HOBt und 0,39 ml N-Ethylmorpholin in 20 ml Dimethylformamid gibt man bei 0°C 660 mg DCC. Aufarbeitung wie bei Beispiel 1a. Die Substanz wird aus Essigester/Petrolether gefällt.
Ausbeute 2,4 g, Schmp. 168°, $[\alpha]_D^{22}$ = -21,4° (c = 1, in Methanol).

### 34b. Indol-3-butyryl-Lys-Tyr-Phe-NH$_2$-acetat, C$_{38}$H$_{48}$N$_6$O$_7$ (MG 700,85)

2,15 g Indol-3-butyryl-Lys(Boc)-Tyr(tBu)-Phe-NH$_2$ werden analog Beispiel 33b umgesetzt und gereinigt.
Ausbeute 402 mg, $[\alpha]_D^{24}$ = -22,9° (c = 1, in Wasser).

### 35. Indol-2-carbonyl-Lys-Tyr-Phe-NH$_2$

### 35a. Indol-2-carbonyl-Lys(Boc)-Tyr(tBu)-Phe-NH$_2$, C$_{42}$H$_{54}$N$_6$O$_7$ (MG 754,9)

485 mg (3 mmol) Indol-2-carbonsäure werden analog Beispiel 34a mit 1,95 g (3 mmol) H-Lys(Boc)-Tyr-(tBu)-Phe-NH$_2$•HCl umgesetzt.
Ausbeute 2,25 g, Schmp. 228-230°C, $[\alpha]_D^{22}$ = -56,7° (c = 1, in Methanol).

### 35b. Indol-2-carbonyl-Lys-Tyr-Phe-NH$_2$-acetat, C$_{35}$H$_{42}$N$_6$O$_7$ (MG 658,7)

2 g Indol-2-carbonyl-Lys(Boc)-Tyr(tBu)-Phe-NH$_2$ werden analog Beispiel 33b umgesetzt.
Ausbeute 1,55 g, $[\alpha]_D^{24}$ = -44,8° (c = 1, in Methanol).

### 36. H-Trp-Arg-Trp-Gly-Trp-Arg-Trp-Gly-OH

### 36a. Fmoc-Trp-Gly-OtBu, C$_{32}$H$_{33}$N$_3$O$_5$ (MG 539,64)

Zu einer Lösung von 21,3 g (50 mmol) Fmoc-Trp-OH, 16,7 g (50 mmol) H-Gly-OtBu•HCl und 7 g HOBt gibt man bei 0°C 11,33 g DCC. Aufarbeitung wie bei Beispiel 1a. Die Verbindung kristallisiert aus Diethylether.
Ausbeute 17,26 g, Schmp. 141°C, $[\alpha]_D^{22}$ = -26,6° (c = 1, in Dimethylformamid).

### 36b. Fmoc-Trp-Arg-OH, $C_{32}H_{34}N_6O_5$ (MG 582,67)

Zu einer Suspension von 6,09 g (35 mmol) Arginin und 6,25 g (35 mmol) Pyridinium-perchlorat in 75 mol Dimethylformamid gibt man 19,98 g (37 mmol) Fmoc-Trp-OObt. Man läßt Rühren bis alles gelöst ist. Danach engt man ein und verrührt den Rückstand mit NaHCO₃-Lösung (pH der Suspension sollte etwa 6 bis 7 sein). Der Niederschlag wird abgesaugt und getrocknet.
Ausbeute 17,65 g, Schmp. 185°C (Zers.), $(\alpha)_D^{22}$ = -24,2° (c = 1, in Dimethylformamid).

### 36c. Fmoc-Trp-Arg-Trp-Gly-OtBu, $C_{49}H_{55}N_{93}O_7$ (MG 882,05)

Zu einer Lösung von 5,4 g (10 mmol) Fmoc-Trp-Gly-OtBu in 25 ml Dimethylformamid gibt man 10,3 ml (100 mmol) Diethylamin. Man läßt 10 Minuten bei Raumtemperatur stehen und engt ein. Der Rückstand wird mit Petrolether mehrmals digeriert. Der Rückstand wird in Diethylether gelöst, von Unlöslichem filtriert und eingeengt. Ausbeute 4,2 g helles Öl (verunreinigt).
Zu einer Lösung von oben gewonnenen 4,2 g Öl, 5,82 g (10 mmol) Fmoc-Trp-Arg-OH und 1,6 g HOObt in 30 ml Dimethylformamid gibt man bei 0°C, 2,2 g DCC. Man läßt über Nacht bei Raumtemperatur stehen, saugt den Niederschlag ab, engt ein und verteilt zwischen Essigester und gesättigter NaHCO₃-Lösung. Die Essigesterlösung wird mit Wasser ausgeschüttelt und eingeengt. Der Rückstand wird mit Diethylether verrieben, abgesaugt und getrocknet.
Ausbeute 8,48 g, Schmp. 145-152°C, $[\alpha]_D^{23}$ = -22,4° (c = 1, in Dimethylformamid).

### 36d. Fmoc-Trp-Arg-Trp-Gly-OH

2,64 g (3 mmol) Fmoc-Trp-Arg-Trp-Gly-OtBu werden analog Beispiel 2c umgesetzt. Der Rückstand wird mit Methyl-tert.-butylether verrieben und abgesaugt. Nach dem Trocknen wird die Substanz mit NaHCO₃-Lösung verrührt (pH der Suspension 6 bis 7). Die Substanz wird abgesaugt und getrocknet
Ausbeute 1,82 g, Schmp. 190-192°C (Zers.), $[\alpha]_D^{22}$ = -43,7° (c = 1, in Dimethylformamid).

### 36e. Fmoc-Trp-Arg-Trp-Gly-Trp-Arg-Trp-Gly-OtBu

Eine Lösung von 2,64 g (3 mmol) Fmoc-Trp-Arg-Trp-Gly-OtBu werden mit 3,3 ml Diethylamin versetzt. Nach 5 Minuten bei Raumtemperatur wird eingeengt und der Rückstand mit Diethylether verrieben und abgesaugt. Ausbeute 2,04 g H-Trp-Arg-Trp-Gly-OtBu. Zu einer Lösung von 1,81 g (2,2 mmol) Fmoc-Trp-Arg-Trp-Gly-OH, 1,45 g (2,2 mmol) H-Trp-Arg-Trp-Gly-OtBu, 0,9 g (2,2 mmol) Pyridinium-perchlorat und 0,36 g (2,2 mmol) HOObt in 25 ml Dimethylformamid gibt man bei 0°C 0,51 g (2,5 mmol) DCC. Man läßt über Nacht bei Raumtemperatur stehen, saugt den Niederschlag ab und engt das Filtrat ein. Der Rückstand wird mit gesättigter NaHCO₃-Lösung verrieben, abgesaugt und mit Wasser gewaschen.
Ausbeute 3,3 g, Schmp. 204-205°C (Zers.), $[\alpha]_D^{22}$ = -27,6° (c = 1, in Dimethylformamid).

### 36f. H-Trp-Arg-Trp-Gly-Trp-Arg-Trp-Gly-OH-diacetat, $C_{64}H_{80}N_{18}O_{13}$ (MG 1309,5)

Zu einer Lösung von 2,93 g (2 mmol) Fmoc-Trp-Arg-Trp-Gly-Trp-Arg-Trp-Gly-OH in 20 ml Dimethylformamid gibt man 2,1 ml (20 mmol) Diethylamin. Man läßt 5 Minuten bei Raumtemperatur stehen und engt ein. Der Rückstand wird mit Diethylether verrieben. Ausbeute 2,7 g H-Trp-Arg-Trp-Gly-Trp-Arg-Trp-Gly-OH.
1 g oben gewonnener Substanz werden analog Beispiel 2c umgesetzt. Reinigung der Substanz an einem alkylierten Dextrangel. Ausbeute 210 mg. Charakterisierung durch UV: charakteristische Bande für Trp bei 280 nm; Aminosäureanalyse in 6 N HCl: 2,00 Gly, 2,04 Arg; Gehalt an Peptidbase: 70 %, $[\alpha]_D^{22}$ = + 1° (c = 1, in 90 %iger Essigsäure).

### 37. cyclo-(Trp-Arg-Trp-Gly-Trp-Arg-Trp-Gly)-diacetat, $C_{64}H_{78}N_{18}O_{14}$ (MG 1291,4)

1,6 g H-Trp-Arg-Trp-Gly-Trp-Arg-Trp-Gly-OtBu werden bei Raumtemperatur in einer Mischung aus 16 ml 90 %iger Trifluoressigsäure und 1,4 ml 1,2-Dimercaptoethan gelöst. Nach 1 Stunde wird eingeengt und der Rückstand mit Methyl-tert.-butylether verrieben und abgesaugt. Ausbeute 1,42 g. Das trockene so gewonnene Material wird mit einer gesättigten NaHCO$_3$-Lösung verrührt (pH der Suspension bei 7), abgesaugt und getrocknet. Ausbeute 0,92 g H-Trp-Arg-Trp-Gly-Trp-Arg-Trp-Gly-OH-ditrifluoracetat.

Zu einer Lösung von oben gewonnener Substanz und 0,185 g HOBt in 6 ml Dimethylformamid gibt man bei 0°C 0,2 g DCC. Man läßt über Nacht bei Raumtemperatur stehen. Anderntags wird der Niederschlag abgesaugt, das Filtrat eingeengt und der Rückstand mit NaHCO$_3$-Lösung verrieben, abgesaugt und getrocknet. Ausbeute 1,05 g. Man löst nun in einer Mischung aus 10 ml Eisessig und 40 ml Wasser und chromatographiert über einen schwach basischen Ionenaustauscher in Acetat-Form. Das Eluat wird eingeengt. Ausbeute 0,83 g. Zur Reinigung wird über ein alkyliertes Dextrangel in einer Mischung aus Methanol/Wasser/Essigsäure wie 50:50:3 chromatographiert. Ausbeute 101 mg. Charakterisierung durch UV: charakteristische Bande für Trp bei 280 nm; Aminosäureanalyse in 6 N HCl: 2,00 Gly, 1,9 Arg; Gehalt an Peptidbase: 82 %.

### 38. Z-Trp-D-Lys-Tyr-Ile-Lys-Pro-OBzl

### 38a. Fmoc-Ile-Lys(Boc)-OH, $C_{32}H_{43}N_3O_7$ (MG 581,7)

Zu einer Suspension von 19,76 g H-Lys(Boc)-OH in 120 ml Dimethylformamid gibt man 40 g Fmoc-Ile-OObt. Man rührt bis alles gelöst ist und engt dann ein. Der Rückstand wird zwischen Essigester und halbgesättigter NaHCO$_3$-Lösung verteilt. Die Essigesterphase wird noch 2mal mit der NaHCO$_3$-Lösung, 2mal mit KHSO$_4$-Lösung und 1mal mit Wasser ausgeschüttelt, über Na$_2$SO$_4$ getrocknet und eingeengt. Die Substanz ist noch mit Fmoc-Ile-OH verunreinigt und wird deshalb 5 Minuten mit 100 ml Methyl-tert.-butylether auf dem Dampfbad erhitzt und abgesaugt.
Ausbeute 28,85 g, Schmp. 162-164°, $[\alpha]_D^{23} = -15,5°$ (c = 1, in Methanol).

### 38b. Fmoc-Ile-Lys(Boc)-Pro-OBzl, $C_{44}H_{56}N_4O_8$ (MG 768,9)

Zu einer Lösung von 28 g Fmoc-Ile-Lys(Boc)-OH, 11,6 g H-Pro-OBzl•HCl, 7,8 g HOObt und 6,3 ml N-Ethylmorpholin in 250 ml Dimethylformamid gibt man bei 0°C 10,6 g DCC. Aufarbeitung wie bei Beispiel 1a. Es bleibt ein Öl zurück, das über Kieselgel in Methylenchlorid/Methanol/Wasser wie 9:1:0,1 chromatographiert wird.
Ausbeute: 30,4 g gelbliches Öl.

### 38c. H-Ile-Lys(Boc)-Pro-OBzl, $C_{29}H_{46}N_4O_5$ (MG 546,7)

Zu einer Lösung von 28,86 g Fmoc-Ile-Lys(Boc)-Pro-OBzl in 200 ml Dimethylformamid gibt man 39 ml Diethylamin. Man läßt 10 Minuten bei Raumtemperatur stehen, engt ein und chromatographiert den Rückstand über Kieselgel in Methylenchlorid und später in Methylenchlorid/Methanol wie 9: 1. Ausbeute 18,4 g gelbliches Öl.

### 38d. Fmoc-D-Lys(Boc)-Tyr(tBu)-OH, $C_{39}H_{49}N_3O_8$ (MG 687,8)

Zu einer Suspension von 11,6 g H-Tyr(tBu)-OH in 150 ml Dimethylformamid gibt man 30 g Fmoc-D-Lys(Boc)-OObt. Man verfährt wie bei Beispiel 38a. Zur Reinigung wird das Peptid 2mal aus Methyl-tert.-butylether/Petrolether gefällt.
Ausbeute 16,45 g, Schmp. 92-94°C, $[\alpha]_D^{23} = -12,8°$ (c = 1, in Methanol).

### 38e. Fmoc-D-Lys(Boc)-Tyr(tBu)-Ile-Lys(Boc)-Pro-OBzl, $C_{68}H_{93}N_7O_{13}$ (MG 1216,5)

Zu einer Lösung von 16,1 g Fmoc-D-Lys(Boc)-Tyr(tBu)-OH, 12,8 g H-Ile-Lys(Boc)-Pro-OBzl und 3,8 g HOObt in 150 ml Dimethylformamid gibt man 5,15 g DCC. Nach etwa 4 Stunden Reaktionszeit bei Raumtemperatur wird der Niederschlag abgesaugt und das Filtrat eingeengt. Der Rückstand wird nacheinander mit Essigester, halbgesättigter NaHCO₃-Lösung und Wasser verrieben und abgesaugt. Aus Isopropanol/Petrolether wird umgefällt.
Ausbeute 18,9 g, Schmp. 147-149°C, $[\alpha]_D^{23} = -31,7°$ (c = 1, in Methanol).

### 38f. H-D-Lys(Boc)-Tyr(tBu)-Ile-Lys(Boc)-Pro-OBzl, $C_{53}H_{83}N_7O_{11}$ (MG 994,3)

Zu einer Lösung von 18 g (14,8 mmol) Fmoc-D-Lys(Boc)-Tyr(tBu)-Ile-Lys(Boc)-Pro-OBzl in 150 ml Dimethylformamid gibt man bei Raumtemperatur 15,4 ml Diethylamin. Nach 20 Minuten wird eingeengt und der Rückstand an Kieselgel chromatographiert. Die Verunreinigungen werden mit Methylenchlorid/Methanol wie 98:2 entfernt und die Substanz mit Methylenchlorid/Methanol wie 90:10 eluiert.
Ausbeute 10,4 g gelbliches Öl.

### 38g. Z-Trp-D-Lys(Boc)-Tyr(tBu)-Ile-Lys(Boc)-Pro-OBzl, $C_{72}H_{93}N_9O_{14}$ (MG 1308,6)

Zu einer Lösung von 10,4 g H-D-Lys(Boc)-Tyr(tBu)-Ile-Lys(Boc)-Pro-OBzl, 3,54 g Z-Trp-OH und 1,41 g HOBt in 150 ml Dimethylformamid gibt man bei 0°C 2,3 g DCC. Aufarbeitung wie bei Beispiel 1a. Der Rückstand wird aus Diethylether/Petrolether gefällt.
Ausbeute 12,65 g, Schmp. 111-113°C, $[\alpha]_D^{23} = -26,4°$ (c = 1, in Methanol).

### 38h. Z-Trp-D-Lys-Tyr-Ile-Lys-Pro-OBzl-diacetat, $C_{62}H_{83}N_9O_{14}$ (MG 1178,4)

1,31 g Z-Trp-D-Lys(Boc)-Tyr(tBu)-Ile-Lys(Boc)-Pro-OBzl werden analog Beispiel 2c umgesetzt. Zur Reinigung wird aus Isopropanol/Petrolether umgefällt.
Ausbeute 892 mg, $[\alpha]_D^{22} = -31,6°$ (c = 1, in Wasser).

### 39. cyclo-(Trp-D-Lys-Tyr-Ile-Lys-Pro)

### 39a. H-Trp-D-Lys(Boc)-Tyr(tBu)-Ile-Lys(Boc)-Pro-OH, $C_{57}H_{87}N_9O_{12}$ (MG 1090,4)

10,5 g Z-Trp-D-Lys(Boc)-Tyr(tBu)-Ile-Lys(Boc)-Pro-OBzl werden in 90 %iger Essigsäure katalytisch hydriert. Der Rückstand wird zwischen Wasser und Essigester verteilt. Die Essigesterphase wird über Na₂SO₄ getrocknet und eingeengt. Der Rückstand wird mit Diethylether verrieben und abgesaugt. Ausbeute 7,0 g. Zur Entfernung anhaftender Essigsäure wird mit NaHCO₃-Lösung bei pH 7 verrührt.
Ausbeute 7,0 g, Schmp. 174-176°C, $[\alpha]_D^{23} = -18,9°$ (c = 1, in Methanol).

### 39b. cyclo-(Trp-D-Lys(Boc)-Tyr-(tBu)-Ile-Lys(Boc)-Pro), $C_{57}H_{85}N_9O_{11}$ (MG 1072,4)

Zu einer Lösung von 1,09 g H-Trp-D-Lys(Boc)-Tyr(tBu)-Ile-Lys(Boc)-Pro-OH und 270 mg HOBt in 200 ml Dimethylformamid gibt man eine Lösung von 440 mg DCC in 100 ml Dimethylformamid. Nach zwei Tagen bei Raumtemperatur gibt man nochmals 135 mg DCC zu und läßt nochmals 2 Tage bei Raumtemperatur reagieren. Danach wird eingeengt und der Rückstand mit Wasser und $NaHCO_3$-Lösung verrieben und abgesaugt. Die Substanz wird an Kieselgel in Methylenchlorid/Methanol/Essigsäure/Wasser wie 150:8:1:1 chromatographiert.
Ausbeute 500 mg

### 39c. cyclo-(Trp-D-Lys-Tyr-Ile-Lys-Pro)-diacetat, $C_{47}H_{69}N_9O_{11}$ (MG 936,12)

500 mg cyclo-(Trp-D-Lys(Boc)-Tyr(tBu)-Ile-Lys-Pro) werden analog Beispiel 2c umgesetzt. Ausbeute 234 mg. Zur Reinigung chromatographiert man an einem alkylierten Dextrangel.
Ausbeute 109,5 mg. Aminosäureanalyse (Hydrolyse in 6N HCl, 24 Stunden bei 120°C): 0,98 Pro, 1,01 Ile, 2,00 Lys, 1,04 Tyr; UV-Spektrum: charakteristische Bande bei 280 nm für Trp.

### 40. Z-Trp-Lys-Tyr-benzylamid

### 40a. Z-Trp-Lys(Boc)-OH, $C_{30}H_{38}N_4O_7$ (MG 566,7)

Zu einer Suspension von 18,45 g (75 mmol) H-Lys(Boc)-OH in 120 ml Dimethylformamid gibt man 38,64 g (80 mmol) Fmoc-Trp-OObt. Man rührt bis alles gelöst ist. Danach wird eingeengt, der Rückstand in Essigester gelöst und mit $NaHCO_3$-Lösung, $KHSO_4$-Lösung und Wasser ausgeschüttelt. Die Essigesterphase wird über $Na_2SO_4$ getrocknet und eingeengt. Der Rückstand wird über Kieselgel chromatogrpahiert. Zur Entfernung von Verunreinigungen wird zuerst mit Methylenchlorid/Aceton wie 9:1 und zur Elution der Substanz mit Methylenchlorid/Methanol wie 95:5 gespült.
Ausbeute 30 g, Schmp. 95-97°C, $[\alpha]_D^{22}$ = -16,4° (c = 1, in Dimethylformamid).

### 40b. Z-Trp-Lys(Boc)-Tyr-OMe, $C_{40}H_{49}N_5O_9$ (MG 743,9)

Zu einer Lösung von 16,68 g (30 mmol) Z-Trp-Lys(Boc)-OH, 6,95 g (30 mmol) H-Tyr-OMe•HCl und 4,92 g (30 mmol) HOObt in 120 ml Dimethylformamid gibt man bei 0°C 3,84 ml N-Ethylmorpholin und 6,8 g DCC. Aufarbeitung wie bei Beispiel 1a. Der Rückstand wird mit Petrolether verrieben und im Hochvakuum getrocknet.
Ausbeute 25,3 g amorphe Substanz.

### 40c. Z-Trp-Lys(Boc)-Tyr-OH, $C_{39}H_{47}N_5O_9$ (MG 729,8)

Zu einer Lösung von 25 g Z-Trp-Lys(Boc)-Tyr-OMe in 120 ml Dioxan gibt man 70 ml Wasser und 35 ml 1N NaOH. Nach 6 Stunden Rühren bei Raumtemperatur wird das Dioxan abdestilliert und mit $KHSO_4$-Lösung auf pH 2 angesäuert. Das sich ausscheidende Öl wird in Essigester eingeschüttelt. Die Essigesterphase wird über $Na_2SO_4$ getrocknet und eingeengt. Das resultierende Öl wird über Kieselgel in Methylenchlorid/Methanol/Wasser/Essigsäure wie 110:10:1:1 chromatographiert.
Ausbeute 13,3 g amorphe Substanz, Schmp. 142-150°C (Zers.), $[\alpha]_D^{22}$ = -12,2° (c = 1, in Dimethylformamid).

### 40d. Z-Trp-Lys(Boc)-Tyr-benzylamid, $C_{46}H_{54}N_6O_8$ (MG 819)

Zu einer Lösung von 2,19 g (3 mmol) Z-Trp-Lys(Boc)-Tyr-OH und 0,492 HOObt in 20 ml Dimethylformamid gibt man 0,33 ml (3 mmol) Benzylamin und bei 0°C 0,68 g DCC. Aufarbeitung wie bei Beispiel 1a. Aus Methanol/Wasser wird umgefällt.
Ausbeute 1,5 g, Schmp. 218-221°C (Zers.), $[\alpha]_D^{22} = -25{,}5°$ (c = 1, in Dimethylformamid).

### 40e. Z-Trp-Lys-Tyr-benzylamid-acetat, $C_{43}H_{50}N_6O_8$ (778,9)

1,3 g Z-Trp-Lys(Boc)-Tyr-benzylamid werden analog Beispiel 2c umgesetzt. Ausbeute 0,92 g. Reinigung durch Chromatographie an Kieselgel in Methylenchlorid/Methanol/Wasser/Essigsäure wie 130:30:4:2.
Ausbeute 335 mg, $[\alpha]_D^{22} = -6{,}1°$ (c = 1, in 90 %iger Essigsäure).

### 41. Z-Trp-Lys-Tyr-phenethylamid

### 41a. Z-Trp-Lys(Boc)-Tyr-phenethylamid, $C_{47}H_{56}N_6O_8$ (MG 833,0)

Herstellung analog Beispiel 40d mit 0,38 ml (3 mmol) Phenethylamin.
Ausbeute 1,48 g, Schmp. 215-218°C (Zers.), $[\alpha]_D^{22} = -23{,}6°$ (c = 1, in Dimethylformamid).

### 41b. Z-Trp-Lys-Tyr-phenethylamid-acetat, $C_{44}H_{52}N_6O_8$ (MG 792,9)

1,3 g Z-Trp-Lys(Boc)-Tyr-phenethylamid werden analog Beispiel 40e umgesetzt und gereinigt.
Ausbeute 438 g, $[\alpha]_D^{22} = -3{,}9°$ (c = 1, in 90 %iger Essigsäure).

### 42. Z-Trp-Lys-Tyr-Phe-ol

### 42a. Z-Trp-Lys(Boc)-Tyr-Phe-ol, $C_{48}H_{58}N_6O_9$ (MG 863,0)

Herstellung analog Beispiel 40d mit 0,95 g (3 mmol) H-Phe-ol•HOObt.
Ausbeute 1,47 g, Schmp. 194-196°C (Zers.), $[\alpha]_D^{22} = -44{,}9°$ (c = 1, in Dimethylformamid).

### 42b. Z-Trp-Lys-Tyr-Phe-ol-acetat, $C_{45}H_{54}N_6O_9$ (MG 822,9)

1,3 g Z-Trp-Lys(Boc)-Tyr-Phe-ol werden analog Beispiel 40e umgesetzt und gereinigt.
Ausbeute 423mg, $[\alpha]_D^{22} = -18{,}8°$ (c = 1, in 90 %iger Essigsäure).

### 43. Z-Trp-Lys-Tic-NH$_2$

34

### 43a. Z-Trp-Lys(Boc)-Tic-NH$_2$, C$_{40}$H$_{44}$N$_6$O$_7$ (MG 720,8)

Zu einer Lösung von 0,99 g (1,75 mmol) Z-Trp-Lys(Boc)-OH, 0,6 g H-Tic-NH$_2$-tosylat und 0,29 g HOObt in 10 ml Dimethylformamid gibt man bei 0°C 0,23 ml N-Ethylmorpholin und 0,38 g DCC. Aufarbeitung analog Beispiel 1a. Die Substanz wird aus Essigester/Petrolether umgefällt.
Ausbeute 1,05 g, Schmp. 160-175°C (Zers.), $[\alpha]_D^{22} = -14,3°$ (c = 1, in Dimethylformamid).

### 43b. Z-Trp-Lys-Tic-NH$_2$-acetat, C$_{37}$H$_{44}$N$_6$O$_7$ (684,8)

0,85 g Z-Trp-Lys(Boc)-Tic-NH$_2$ werden analog Beipsiel 40e umgesetzt und gereinigt.
Ausbeute 195 mg, $[\alpha]_D^{24} = -24,1°$ (c = 1, in 90 %iger Essigsäure).

### 44. Z-D-Phe-D-Tyr-D-Lys-D-Trp-NH$_2$

### 44a. Z-D-Lys(Boc)-D-Trp-NH$_2$, C$_{30}$H$_{39}$N$_5$O$_6$ (MG 565,7)

Zu einer Lösung von 4,3 g (18 mmol) H-D-Trp-NH$_2$•HCl, 2,52 g (18 mmol) HOBt und 7,22 g Z-D-Lys-(BOC)-OH in 75 ml Dimethylformamid gibt man bei 0°C 2,3 ml N-Ethylmorpholin und 3,91 g DCC. Aufgearbeitet wird analog Beispiel 1a. Der Rückstand wird mit Methyl-tert.-butylether verrieben.
Ausbeute 9,05 g, Schmp. 200-205°C (Zers.), $[\alpha]_D^{22} = -11,2°$ (c = 1, in Dimethylformamid).

### 44b. H-D-Lys(Boc)-D-Trp-NH$_2$, C$_{22}$H$_{34}$Cl$_1$N$_5$O$_4$ (MG 468,0)

8,75 g Z-D-Lys(Boc)-D-Trp-NH$_2$ werden analog Beispiel 2a katalytisch hydriert.
Ausbeute 7,25 g, 170-185°C (Zers.), $[\alpha]_D^{22} = -1,7°$ (c = 1, in Dimethylformamid).

### 44c. Z-D-Tyr(tBu)-D-Lys(Boc)-D-Trp-NH$_2$, C$_{43}$H$_{56}$N$_6$O$_8$ (MG 784,9)

Zu einer Lösung von 7,02 g (15 mmol) H-D-Lys(Boc)-D-Trp-NH$_2$•HCl, 6 g (16 mmol) Z-D-Tyr(tBu)-OH und 2,1 g HOBt in 75 ml Dimethylformamid gibt man bei 0°C 1,92 ml (15 mmol) N-Ethylmorpholin und 3,5 g DCC. Aufarbeitung wie bei Beispiel 1a. Rückstand mit Diethylether verreiben.
Ausbeute 8,54 g, Schmp. 178-185°C (Zers.), $[\alpha]_D^{22} = 10,8°$ (c = 1, in Dimethylformamid).

### 44d. H-D-Tyr(tBu)-D-Lys(Boc)-D-Trp-NH$_2$•HCl, C$_{35}$H$_{51}$Cl$_1$N$_6$O$_6$ (MG 687,3)

8,3 g (10,5 mmol) Z-D-Tyr(tBu)-D-Lys(Boc)-D-Trp-NH$_2$ werden analog Beispiel 2a katalytisch hydriert.
Ausbeute 7,14 g, Schmp. 190-192°C (Zers.), $[\alpha]_D^{22} = -11,2°$ (c = 1, in Dimethylformamid).

### 44e. Z-D-Phe-D-Tyr(tBu)-D-Lys(Boc)-D-Trp-NH$_2$, C$_{52}$H$_{65}$N$_7$O$_9$ (MG 932,1)

Zu einer Lösung von 2,74 g (4 mmol) H-D-Tyr(tBu)-D-Lys(Boc)-D-Trp-NH$_2$•HCl, 1,21 g (4 mmol) Z-D-Phe-OH und 0,66 g HOObt in 25 ml Dimethylformamid gibt man bei 0°C 0,52 ml N-Ethylmorpholin und 1,1 g DCC. Aufarbeitung wie bei Beispiel 1a.
Ausbeute 2,77 g, Schmp. 179-185°C (Zers.), $[\alpha]_D^{22} = 20,1°$ (c = 1, in Dimethylformamid).

35

**44f. Z-D-Phe-D-Tyr-D-Lys-D-Trp-NH₂-acetat, $C_{45}H_{53}N_7O_9$, (MG 836)**

1 g Z-D-Phe-D-Tyr(tBu)-D-LyS(Boc)-D-Trp-NH₂ werden analog Beispiel 2c umgesetzt. Ausbeute 434,1 mg, $[\alpha]_D^{22} = 4,9°$ (c = 1, in 90 %iger Essigsäure).

### Verwendete Abkürzungen:

Die für Aminosäuren verwendeten Abkürzungen entsprechen dem in der Peptidchemie üblichen Drei-Buchstaben-Code wie er z.B. in Europ. J. Biochem. 138 9 (1984) beschrieben ist. Einige weitere Abkürzungen sind nachfolgend aufgelistet:

Ac-    Acetyl
Boc-    tert.-Butyl-oxycarbonyl-
DCC    Dicyclohexylcarbodiimid
Et-    Ethyl
Fmoc-    9-Fluorenyl-methyl-oxycarbonyl-
HOBt    1-Hydroxy-benzotriazol
HOObt    3-Hydroxy-4-oxo-3.4-dihydro-1.2.3-benzotriazol
-ol    eine zum Alkohol reduzierte α-Carboxylgruppe eines C-terminalen Aminosäure-restes
-OMe    Methyl-ester
-ONSu    N-Hydroxysuccinimid-ester
-OtBu    tert.-Butyl-ester
-OTcp    2.3.4-Trichlorphenylester
-StBu    tert.-Butylmercapto
-tBu    tert.-Butyl
Tic    Tetrahydroisochinolin-3-carbonsäure
Z-    Benzyl-oxycarbonyl-

### Ansprüche

1. Peptid, deren C-terminale α-Carboxylgruppe durch eine Esterfunktion oder eine Amidfunktion geschützt ist, der allgemeinen Formel I

L - B - A    (I),

in welcher

L    fehlen kann oder einen lipophilen Rest,
B    eine basische Aminosäure und
A    eine aromatische Aminosäure bedeuten,

sowie dessen physiologisch verträgliche Salze.

2. Peptid der allgemeinen Formel I gemäß Anspruch 1, worin L eine N-terminal durch lipophile Acyl-oder Peptidreste geschützte lipophile Aminosäure in der L-oder D-Konfiguration, die acylartig an die α-Aminogruppe der basischen Aminosäure gebunden ist, bedeutet, oder für einen lipophilen Acylrest oder einen ($C_1$-$C_{17}$)-Alkylcarbonyl-Rest steht, sowie dessen physiologisch verträgliche Salze.

3. Peptid der allgemeinen Formel I gemäß Anspruch 1, worin

L    Isoleucin, Leucin, Methionin, S-geschütztes-Cystein, Valin; gegebenenfalls durch Halogen, ($C_1$-$C_4$)-Alkyl oder ($C_1$-$C_4$)-Alkoxy substituiertes Phenylalanin, Tryptophan oder Naphthylalanin; an der Hydroxy-gruppe durch ($C_1$-$C_4$)-Alkyl substituiertes Tyrosin, Serin oder Threonin; am N des Imidazolringes durch ($C_1$-$C_4$)-Alkyl substituiertes Histidin; in der Seitenkette durch ($C_1$-$C_4$)-Alkyl veresterte oder durch liphophile Amine amidierte Asparaginsäure, Glutaminsäure oder Aminoadipinsäure; oder an der ω-Aminogruppe durch ($C_1$-$C_4$)-Alkylcarbonyl substituiertes bzw. unsubstituiertes Lysin oder Ornithin; lipophile Reste oder ($C_1$-$C_{17}$)-

Alkylcarbonyl;

B       gegebenenfalls an der Guanidogruppe alkyliertes $(C_1-C_6)$-Arginin oder -Homoarginin, Lysin, Ornithin, Hydroxylysin oder Citrullin in der D-oder L-Konfiguration und

A       gegebenenfalls im Aromaten durch 1 bis 5 gleiche oder verschiedene Reste aus der Reihe Halogen, $(C_1-C_4)$-Alkyl oder $(C_1-C_4)$-Alkoxy substituiertes Phenylalanin, Tryptophan, Naphthylalanin, Thienylalanin oder Tetrahydro-isochinolin-3-carbonsäure; oder gegebenenfalls an der Hydroxygruppe durch $(C_1-C_4)$-Alkyl substituiertes Tyrosin jeweils in der L-oder D-Konfiguration, deren Carboxylgruppen durch $(C_1-C_4)$-Alkyl- oder $(C_5-C_{14})$-Aryl-$(C_1-C_4)$-alkylester, durch die Amidgruppe, durch primäre $(C_1-C_4)$-Alkyl-, $(C_5-C_{14})$-Aryl-$(C_1-C_4)$-alkylamide und Peptidreste, bestehend aus bis zu 4 natürlich vorkommenden Aminosäuren, geschützt sind bedeuten,

sowie dessen physiologisch verträgliche Salze.

4. Verfahren zur Herstellung eines Peptids der Formel I gemäß einem oder mehreren der Ansprüche 1 bis 3, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abgespaltet und die so erhaltene Verbindung in ihr physiologisch verträgliches Salz überführt.

5. Verwendung eines Peptids der Formel I gemäß einem oder mehreren der Ansprüche 1 bis 3 als Heilmittel.

6. Verwendung eines Peptids der Formel I gemäß einem oder mehreren der Ansprüche 1 bis 3 zur Behandlung entzündlicher, allergischer, rheumatischer und autoimmunologischer Erkrankungen.

7. Verwendung eines Peptids der Formel I gemäß einem oder mehreren der Ansprüche 1 bis 3 zur Behandlung von Bluterkrankungen, Hauterkrankungen sowie Abstoßungsreaktionen nach Transplantationen.

8. Pharmazeutisches Mittel, enthaltend ein Peptid der Formel I gemäß einem der Ansprüche 1 bis 3.

9. Verfahren zur Herstellung eines pharmazeutischen Mittels gemäß Anspruch 8, dadurch gekennzeichnet, daß man ein Peptid der allgemeinen Formel I oder dessen physiologisch verträgliches Salz zusammen mit einem physiologisch annehmbaren Träger und gegebenenfalls weiteren Zusatz-, Hilfs-und/oder Konservierungsstoffen in eine geeignete Darreichungsform bringt.

Patentansprüche für folgende Vertragsstaaten : ES, GR

1. Verfahren zur Herstellung eines Peptids, deren C-terminale $\alpha$-Carboxylgruppe durch eine Esterfunk- tion oder eine Amidfunktion geschützt ist, der allgemeinen Formel I

L - B - A      (I),

in welcher

L      fehlen kann oder einen lipophilen Rest,

B      eine basische Aminosäure und

A      eine aromatische Aminosäure bedeuten,

sowie dessen physiologisch verträgliche Salze, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abgespaltet und die so erhaltene Verbindung in ihr physiologisch verträgliches Salz überführt.

2. Verfahren gemäß Anspruch 1, dadurch gekennzeichnet, daß eine Verbindung der allgemeinen Formel I hergestellt wird, worin L eine N-terminal durch lipophile Acyl-oder Peptidreste geschützte lipophile Aminosäure in der L-oder D-Konfiguration, die acylartig an die $\alpha$-Aminogruppe der basischen Aminosäure gebunden ist, bedeutet, oder für einen lipophilen Acylrest oder einen $(C_1-C_{17})$-Alkylcarbonyl-Rest steht, sowie dessen physiologisch verträgliche Salze.

3. Verfahren gemäß Anspruch 1, dadurch gekennzeichnet, daß ein Peptid der allgemeinen Formel I hergestellt wird, worin

L       Isoleucin, Leucin, Methionin, S-geschütztes-Cystein, Valin; gegebenenfalls durch Halogen, $(C_1-C_4)$ Alkyl oder $(C_1-C_4)$-Alkoxy substituiertes Phenylalanin, Tryptophan oder Naphthylalanin; an der Hydroxygruppe durch (C₁-C₄)-Alkyl substituiertes Tyrosin, Serin oder Threonin; am N des Imidazolringes durch (C₁-C₄)-Alkyl substituiertes Histidin; in der Seitenkette durch (C₁-C₄)-Alkyl veresterte oder durch liphophile Amine amidierte Asparaginsäure, Glutaminsäure oder Aminoadipinsäure; oder an der ω-Aminogruppe durch (C₁-C₄)-Alkylcarbonyl substituiertes bzw. unsubstituiertes Lysin oder Ornithin; lipophile Reste oder (C₁-C₁₇) Alkylcarbonyl;

B gegebenenfalls an der Guanidogruppe alkyliertes (C₁-C₆)-Arginin oder -Homoarginin, Lysin, Ornithin, Hydroxylysin oder Citrullin in der D-oder L-Konfiguration und

A gegebenenfalls im Aromaten durch 1 bis 5 gleiche oder verschiedene Reste aus der Reihe Halogen, (C₁-C₄)-Alkyl oder (C₁-C₄)-Alkoxy substituiertes Phenylalanin, Tryptophan, Naphthylalanin, Thienylalanin oder Tetrahydro-isochinolin-3-carbonsäure; oder gegebenenfalls an der Hydroxygruppe durch (C₁-C₄)-Alkyl substituiertes Tyrosin jeweils in der L-oder D-Konfiguration, deren Carboxylgruppen durch (C₁-C₄)-Alkyl- oder (C₅-C₁₄)-Aryl-(C₁-C₄)-alkylester, durch die Amidgruppe, durch primäre (C₁-C₄)-Alkyl-, (C₅-C₁₄)-Aryl-(C₁-C₄)-alkylamide und Peptidreste, bestehend aus bis zu 4 natürlich vorkommenden Aminosäuren, geschützt sind bedeuten,

sowie dessen physiologisch verträgliche Salze.

4. Verfahren zur Herstellung eines pharmazeutischen Mittels enthaltend ein Peptid der allgemeinen Formel I gemäß Anspruch 1, dadurch gekennzeichnet, daß man dieses oder dessen physiologisch verträgliches Salz zusammen mit einem physiologisch annehmbaren Träger und gegebenenfalls weiteren Zusatz-, Hilfs-und/oder Konservierungsstoffen in eine geeignete Darreichungsform bringt.